(19) Europäisches Patentamt
European Patent Office
Office européen des brevets

(11) **EP 2 153 227 B1**

(12) **EUROPEAN PATENT SPECIFICATION**

(45) Date of publication and mention
of the grant of the patent:
**16.02.2011 Bulletin 2011/07**

(51) Int Cl.:
***G01N 33/50*** (2006.01)

(21) Application number: **08829343.6**

(22) Date of filing: **28.05.2008**

(86) International application number:
**PCT/IB2008/003187**

(87) International publication number:
**WO 2009/031049 (12.03.2009 Gazette 2009/11)**

(54) **REAGENTS AND METHODS FOR THE DETERMINATION OF PK/ADME-TOX CHARACTERISTICS OF NEW CHEMICAL ENTITIES AND OF DRUG CANDIDATES**

REAGENZIEN UND VERFAHREN ZUR BESTIMMUNG VON PK/ADME-TOX-EIGENSCHAFTEN NEUER CHEMISCHER EINHEITEN UND WIRKSTOFFKANDIDATEN

RÉACTIFS ET PROCÉDÉS DE DÉTERMINATION DES CARACTÉRISTIQUES PK/ADME-TOX D'ENTITÉS CHIMIQUES INÉDITES ET DE CANDIDATS MÉDICAMENTS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.05.2007 US 940641 P**

(43) Date of publication of application:
**17.02.2010 Bulletin 2010/07**

(73) Proprietor: **Pharma Diagnostics NV**
**Z1 Research Park 310**
**1731 Zellik (BE)**

(72) Inventors:
• **ENGLEBIENNE, Patrick**
**B-9750 Zingem (BE)**
• **VAN HOONACKER, Anne**
**B-9750 Zingem (BE)**
• **MARTINEZ-NEIRA, Roberto**
**B-1083 Ganshoren (BE)**
• **DE PRIL, Patricia**
**B-9550 Sint-lievens-esse (BE)**

(74) Representative: **Laenen, Bart Roger Albert et al**
**LC Services BVBA**
**Crutzenstraat 24**
**3500 Hasselt (BE)**

(56) References cited:
**WO-A-2006/047371**

• **ENGLEBIENNE, P. ET AL: "High-throughput screening using the surface plasmon resonance effect of colloidal gold nanoparticles" THE ANALYST, vol. 126, 2001, pages 1645-1651, XP002520452**
• **ENGLEBIENNE, P.: "Use of colloidal gold surface plasmon resonance peak shift to interafinity constants from the interactions between protein antigens and antibodies specific for single or multiple epitopes" THE ANALYST, vol. 123, 1998, pages 1599-1603, XP008015733**
• **ENGLEBIENNE, P. ET AL: "Advances in high-throughput screening: biomolecular interaction monitoring in real-time with colloidal metal nanoparticles" COMBINATORIAL CHEMISTRY & HIGH THROUGHPUT SCREENING, vol. 6, 2003, pages 777-787, XP009035035**

**Description**

INTRODUCTION

It is now widely accepted that the process of converting new chemical entities (NCEs) into marketed drugs is not efficient. Pharmaceutical companies must screen 5-10,000 compounds for every new drug that reaches the market. The most commonly cited estimate suggests that it costs $800 million R&D expenditure to launch one new drug, most of the cost being expended into failures (Bains, W. (2004) Failure rates in drug discovery and development: Will we ever get any better? Drug Discovery World 5(4), 9-18.).

Traditionally, drugs were obtained from natural sources or synthesized, one at a time. In traditional drug development protocols, synthesis of new compounds was considered the rate limiting step of the drug discovery and development process. The advent of automated combinatorial chemistry has circumvented this rate limiting step by making it possible to synthesize thousands of compounds in a single day.

This technological improvement of organic synthesis called rapidly for the implementation of high-throughput screening (HTS) methods giving the pharmaceutical scientists the opportunity to test in a matter of days the biological activity of so many compounds toward pharmaceutical targets, namely cellular receptors, ion channels or enzymes (Englebienne, P. (2005) High-Throughput Screening: Will the past meet the future? In: Frontiers in Drug Design and Discovery, Vol. 1 (G.W. Caldwell, Atta-Uhr-Rahman and B.A. Springer, eds.), Bentham Science Publishers, Ltd, Hilversum, pp. 69-86). However, despite this dramatic increase in synthetic and biological evaluation speed, the number of compounds that survives the full drug development process remains extremely low. The output of new drugs launched per year has remained virtually flat from 1963 to 1999 (Service, R.F. (2004) Surviving the blockbuster syndrome. Science 303, 1796-1799).

The major reasons for the failure of many NCEs are poor pharmacokinetic (PK), ADME (absorption, distribution, metabolism and excretion) and Tox (toxicological) characteristics. These reasons alone account for up to 50% of the attrition rate of new drug candidates during the drug development process. Other reasons of attrition are the lack of efficacy (30%), adverse effects in man (10%), commercial or miscellaneous (10%) (Wang, J. and Urban, L. (2004) The impact of early ADME profiling on drug discovery and development strategy. Drug Discovery World 5(4), 73-86).

The drug discovery and development process may be categorized into three phases: discovery, development and registration. The discovery phase (synthesis, lead finding, lead optimization) may last three to four years. The development phase includes the clinical phases I-III. It is during the late discovery and early development (clinical phase I) that PK/ADME-Tox characteristics are respectively evaluated in cell culture models and animals (Englebienne, 2005, supra). Considering that a clinical phase I usually lasts about two years, most failures occur after two up to six years of research efforts have been spent on an NCE. Consequently, it is more than desirable to obtain critical information about the PK/ADME-Tox characteristics of a drug candidate as early as possible in the drug discovery process.

With an aim at obtaining rapidly such information during the drug discovery and development process, many quantitative structure-activity relationship (QSAR) models have been proposed (Yoshida, F. and Topliss, J.G. (2000) QSAR model for drug human oral bioavailability. J. Med. Chem. 43, 2575-2585; Bergström, C.A.S., Strafford, M. et al. (2003) Absorption classification of oral drugs based on molecular surface properties. J. Med. Chem., 46, 558-570; Sköld, C., Winiwater, S. et al. (2006) Presentation of a structurally diverse and commercially available drug data set for correlation and benchmarking studies. J. Med. Chem., 49, 6660-6671). QSAR models are computer programs that allow one to relate in silico (i.e., by computer-assisted calculations) chemical structure characteristics to a given probability that a molecule bearing such molecular characteristics will display corresponding ADME properties. Several patent and patent applications describing such in silico techniques have been filed. See e.g., United States Published Application Nos. 2004009536 and 2005119832.

In vitro methods have also been developed and published, allowing one to infer the lipophilicity (Lombardo, F., Obach, R.S. et al. (2004) Prediction of human volume of distribution values for neutral and basic drugs. 2. Extended data set and leave-class-out statistics. J. Med. Chem. 47, 1242-1250; Wilson, D.M., Wang, X. Et al. (2001) High-throughput Log D determination using liquid chromatography-mass spectrometry. Combinatorial Chem. High Throughput Screen. 4, 511-519), the membrane permeation potential (Taillardat-Bertschinger, A., Carrupt, P.A. et al. (2003) Immobilized artificial membrane HPLC in drug research. J. Med. Chem. 46, 655-665), or plasma protein-binding (Cimitan, S. Lindgren, M.T. et al. (2005) Early absorption and distribution analysis of antitumor and anti-AIDS drugs: lipid membrane and plasma protein interactions. J. Med. Chem. 48, 3536-3546) of drug candidates, all characteristics that govern their ADME-Tox properties. See also International Patent Publication Nos. WO9942511; WO03027656; and WO2005034915, as well as United States Patent No. US 6,868,343.

Despite the fact that such in vitro methods are usually labeled as being of high-throughput screening nature, their performance and timing are rather low. Many such methods require long incubation times and the separation of bound from free drug must be performed (e.g., by centrifugation), before that the measurement of the concentration of drug either bound to the solid-phase reagent, or free in solution, can respectively take place. Moreover, the drug concentrations are inferred from data obtained by complex technologies including high performance liquid chromatography (HPLC), radioisotopic counting and/or mass spectrometry.

SUMMARY

Embodiments of the present invention circumvent these drawbacks by providing in vitro methods for the determination of ADME characteristics of NCEs based on a "mix and read" technology taking advantage of the photonic reactivity of noble metal colloids, of conductive polymer colloids, or of composite thereof, as described in greater detail below.

Nanoparticles of noble metals such as gold or silver can be prepared in various geometrical forms such as spheres, rods or pyramids. These small objects contain the metallic element in chemically reduced form and depending on the way they are prepared, they can be either stored as reduced powdered solids, or held in stable suspension in solvents such as water or various organic solvents (i.e., as a colloid). Because of the nanometer size of the particles, the naked eye cannot distinguish such suspensions from true solutions, although the microscope can, and such suspensions are therefore termed colloidal solutions. The particles are therefore easily cast on various supports to form well-defined structures.

The study of these metal nanoparticles has been an extremely active area in recent years because of their unique electronic, optical and catalytic properties. Since light is associated with an electromagnetic field, the optoelectronic properties of the nanoparticles are particularly interesting. Indeed, because they are metallic and capable of conducting electricity, the noble metal nanoparticles are surrounded at their surface by a dense cloud of conducting electrons. When these electrons are excited by light, the electromagnetic radiation combines with these electrons to form collective oscillations that radiate away from the particle surface. As a result, the particles exhibit specific light absorption, reflection, emission and scattering properties that can be successfully applied in analytical technology. Indeed, the binding of a ligand to a receptor immobilized on the nanoparticle surface induces a change in the refractive index around the particle which is directly translated by a specific change in its photonic properties (Englebienne, P. et al. (2003) Surface plasmon resonance: Principles, methods and applications in biomedical sciences. Spectroscopy 17, 255-278). Most of the particles studied so far are homogeneously made of the same metal. However, it has been shown recently that it was possible to construct nano-objects made of different metals alternating in the structure. This new advance opens the way to their use as nano bar-codes in numerous applications, including the quantitative detection of biomolecular interactions (Bao, Y.P., Wei, T.F., Lefebvre, P.A. et al. (2006) Detection of protein analytes via nanoparticle-based bio bar code technology. Anal. Chem. 78, 2055-2059).

Similarly, conductive polymers or composites made of conductive polymers and noble metals can also be obtained in colloidal form containing nanoparticles. Conductive polymers are extensively conjugated organic macromolecules made of repetitive sequences of respectively alkyne, phenyl or heterocyclic monomers. These polymers can gain near-metallic conductivity by oxidation (p-doping) or reduction (n-doping) from their insulating state. Another important feature common to the conductive polymers is their capacity to undergo chromic transitions upon changes in their oxidation state (Englebienne, P. (1999) Synthetic materials capable of reporting biomolecular recognition events by chromic transition. J. Mater. Chem. 9, 1043-1054). The use of composite nanoparticles made of a noble metal surrounded by a layer of conductive polymer induces the interaction of the conducting electrons of both materials, which allows for an increase in the photonic reactivity of the nanomaterials to various changes in their environment, including the refractive index and redox potential (Englebienne, P. and Van Hoonacker, A. (2005) Gold-conductive polymer nanoparticles: A hybrid material with enhanced photonic reactivity to environmental stimuli. J. Coll. Interface Sci. 292, 445-454) See also Published United States Patent Application No. 2007-0172834.

Because of the specific photonic sensitivity of such nanoparticles to their environment, they have been used as labels in various homogeneous analytical techniques, including quantitative immunoassays for various analytes (Englebienne, P. (2000) Immune and Receptor Assays in Theory and Practice. CRC Press, Boca Raton, 392pp). A lipid bilayer membrane containing a specific receptor, covalently labeled with a conductive polymer and cast on a solid support, has also been proposed for monitoring directly by spectrophotometry its interaction with the specific ligand such as described in US 6,395,561.

The present invention demonstrates and claims the advantageous application of colloidal nanoparticles made of conductive polymers, noble metals or composites thereof, used in solution or on a solid support, to the rapid determination of PK/ADME-Tox properties of NCEs.

Accordingly, methods of using nanoparticle colloids, such as colloidal conductive polymers, noble metal nanoparticles or of stable metal/conductive polymer composite colloids, either in solution or cast on a solid support, as reagents for at least the prediction of, and in some embodiments the determination of, in vitro, PK/ADME-tox properties of NCEs (e.g., drug candidates or lead compounds), are provided. . Aspects of embodiments of these methods and reagents include the fact that the reactions in which they are involved are homogeneous, rapid, and proceed as "mix and read" processes.

BRIEF DESCRIPTION OF THE FIGURES

Fig. 1 shows the binding isotherm of theophylline with a BSA-gold colloid conjugate.

Fig. 2 shows the binding isotherm of bilirubin with a BSA-gold colloid conjugate.

Fig. 3 shows the titration curve of phosphate-buffered poly(aniline).

Fig. 4 shows the kinetics of association and dissociation of hSA-digoxine.

Fig. 5 shows the ascorbic acid standard curve.

Fig. 6 shows the regression and correlation functions between the apparent volume of distribution obtained with data for paracetamol, acetylsalicylic acid, erythromycin, theophyllin, chlorpheniramine and salicylate, with the volume of distribution obtained from in vivo pharmacokinetics.

Fig. 7 shows the binding isotherm for digoxin with an HSA-gold colloid conjugate.

Fig. 8 shows the binding isotherm for thiopental with an HSA-gold colloid conjugate.

Fig. 9 shows the HSA-gold colloid conjugate assay reproducibility for six known drugs: furosemide, indomethacine, ibuprofen, oxacilline, imipramine, and naproxen.

Fig. 10 shows the correlation between the apparent affinity observed with the HSA-gold colloid conjugate assay and apparent affinity data from the literature (experimental or calculated from % serum protein binding). The dashed line indicates identity.

Fig. 11 shows the binding isotherms for verapamil and erythromycine with a human orosomucoid ($\alpha$1- acid glycoprotein) gold colloid conjugate.

Fig. 12 shows the human orosomucoid ($\alpha$1- acid glycoprotein) gold colloid conjugate assay reproducibility and the correlation of the apparent affinities observed for amitryptiline, chlorpheniramine, chlorpromazine, desipramine, diphenylhydramine HCl, erythromycine, haloperidol, indomethacin, imipramine, phenylbutazone, pindolol, prazosin, prednisolone, propranolol, quinidine, tubocurarine, valproate, verapamil, and warfarin.

Fig 13 shows the correlation obtained for known drugs between the LSPR signal and the Caco-2 permeation rate constant (literature data).

Fig 14 shows the correlation obtained for known drugs between the LSPR signal and the % fraction absorbed in the intestinal tract.

Fig. 15 shows the correlation between the 450 nm signal of PANI-COOH after reaction with known compounds and their respective reduction potentials (literature data).

Fig. 16 shows a titration curve for quinidine with colloidal gold.

Fig. 17 shows the correlation (n = 7; $r^2$ = 0.89) between measured and expected pKa values with colloidal gold.

DEFINITIONS

Unless defined otherwise, all technical and scientific terms used herein have the same meaning as commonly understood by one of ordinary skill in the art to which this invention belongs. Still, certain elements are defined below for the sake of clarity and ease of reference.

The term "colloid" refers to a fluid composition of microscopic particles suspended in a liquid medium. In representative colloids, the particles therein are between one nanometer and one micrometer in size.

The term metal colloid refers to a colloid in which the suspended microscopic particles are metal particles.

The term "noble metal" refers to Group VIII metals of the Periodic Table including, but not limited to: platinum, iridium, palladium and the like, as well as gold, silver etc.

The term "conductive polymer" or "conducting polymer" means an electrically conductive polymeric material. In representative embodiments, conductive polymers are organic polymers, such as $\pi$-conjugated organic polymers. For example, employed may be polypyrrole, polythiophene, polybenzothiophene, polyisothianaphthene, polythienylenevinylene, polyaniline, polyacetylene, polydiacetylene, polyazulene, polypyrene, polycarbazole, polyselenophene, polyfuran and polybenzofuran, poly(p-phenylene), polyindole, polypyridazine, and polyacenes such as naphthacene, pentacene, hexacene, heptacene, dibenzopentacene, tertabenzopentacene, pyrene, dibenzopyrene, chrysene, perylene, coronene, terylene, ovalene, quoterylene, and circumanthracene and their derivatives.

As is known in the art, the conducting polymer may be doped by incorporating into the polymer materials having a functional group such as a dimethylamino group, a cyano group, a carboxyl group and a nitro group, materials such as benzoquinone derivatives, and tetracyanoethylene as well as tetracyanoquinodimethane, and derivatives thereof, which work as an acceptor which accepts electrons, or, for example, materials having a functional group such as an amino group, a triphenyl group, an alkyl group, a hydroxyl group, an alkoxy group, and a phenyl group; substituted amines such as phenylenediamine; anthracene, benzoanthracene, substituted benzoanthracenes, pyrene, substituted pyrene, carbazole and derivatives thereof, and tetrathiafulvalene and derivatives thereof, which work as a donor which is an electron donor. The doping, as described herein, means that electron accepting molecules (acceptors) or electron donating molecules (donors) are incorporated in said polymer employing doping. Employed as dopants used in the present invention may be either acceptors or donors

The term "colloidal conductive polymer' refers to a colloid made up of conductive polymer particles.

The term "metal/conductive polymer composite colloid" refers to a colloid made up of metal particles having a conductive polymer present on a surface thereof.

The terms "colloid conjugate" or "conjugated nanoparticle" refers to a nanoparticle taking part of a colloid on the surface of which a molecule is displayed to confer reactivity, such as, but not limited to a receptor, a nucleic acid, a thiolated alkyl chain,etc. The attachment of the reacting molecule on the surface of the nanoparticle may occur by physical or chemical means.

The terms "solid support" and "solid substrate" mean any light transparent solid-phase such as, but not limited to, glass or quartz, on which the colloids are deposited to form a photo-reactive film to be used as a sensor.

The term "adsorb" refers to the adhesion in a thin layer of molecules (e.g., a receptor, a membrane or ligands) to the surfaces of solid bodies, e.g., metal or composite particles, with which they are in contact.

As used herein, the term "contacting" means to bring or put together. As such, a first item is contacted with a second item when the two items are brought or put together, e.g., by touching them to each other. The term "combining" refers to contacting two different compositions in a manner such that they become a single composition.

The term "agitation" refers to application of physical movement to a composition, such that the components thereof move relative to each other. As such, the term agitation is employed broadly to refer to mixing, stirring, and the like.

The term "ligand" as used herein refers to any type of molecule that is a member of a specific binding pair. Ligands of interest include, but are not limited to biomolecules, where the term "biomolecule" means any inorganic, organic or biochemical molecule, group or species of interest, e.g., that can specifically bind to or interact with a receptor of interest, such as an antibody, a cell receptor, an ion channel, a plasma protein, an enzyme or a lipidic structure. The ligand may also form a covalent adduct with the corresponding binding molecule, such as by oxidative attack of a sulfhydryl group. Exemplary biomolecules include peptides, proteins, amino acids and nucleic acids. For specific embodiments of the present invention, a ligand may be a drug candidate, or any new chemical entity (NCE) synthesized or isolated by pharmaceutical chemists to be developed as a lead compound in the drug discovery and development process.

The term "peptide" as used herein refers to any compound produced by amide formation between a carboxyl group of one amino acid and an amino group of another group.

The term "oligopeptide" as used herein refers to peptides with fewer than about 10 to 20 residues, i.e. amino acid monomeric units.

The term "polypeptide" as used herein refers to peptides with more than about 10 to about 20 residues. The terms "polypeptide" and "protein" may be used interchangeably.

The term "protein" as used herein refers to polypeptides of specific sequence of more than about 50 residue and includes D and L forms, modified forms, etc.

The term "nucleic acid" as used herein means a polymer composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "nucleoside" and "nucleotide" are intended to include those moieties that contain not only the known purine and pyrimidine base moieties, but also other heterocyclic base moieties that have been modified. Such modifications include methylated purines or pyrimidines, acylated purines or pyrimidines, or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The terms "ribonucleic acid" and "RNA" as used herein refer to a polymer composed of ribonucleotides.

The terms "new chemical entity", "drug candidate" and "lead compound" as used herein refer to inorganic or organic compounds, including metal salts, small molecules made of carbon complexes of oxygen, hydrogen, nitrogen, phosphorous, silicon, peptides, proteins and nucleic acids isolated from natural sources or synthesized de novo to be developed as a drug for human or animal use.

By "homogeneous" is meant that a composition is of the same or a similar kind or nature throughout, i.e., of uniform structure or composition throughout.

The term "homogeneous assay" refers to an analytical assay system based on the "mix and read" principle, i.e. which does not require any physical separation of bound and free ligand and the result of which is directly measured from the complete reaction mixture.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

A "biopolymer" is a polymer of one or more types of repeating units. Biopolymers are found in biological systems (although they may be made synthetically) and may include peptides, proteins or polynucleotides, as well as such compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. For example, a "biopolymer" may include receptors, antibodies, binding-proteins, DNA (including cDNA), RNA, oligonucleotides, and PNA and other polynucleotides as described in U.S. Pat. No. 5,948,902 and references cited therein, regardless of the source.

The phrase "optical property" refers to an optical parameter, i.e., a property whose value determines the characteristic or behavior of something, where representative optical properties include, but are not limited to: light absorption, light emission, light reflection and light scattering.

The term "localized surface plasmon resonance" refers to the specific light absorption band in the visible spectrum of noble metal nanoparticles or of conductive polymer composites thereof. As is known by one of ordinary skill in the art, the wavelength and height of such specific absorption band are respectively shifted and increased when the refractive index (i.e. the square root of the dielectric) of the surrounding medium is modified.

By "surface plasmon resonance coupling" is meant the increase in the localized surface plasmon resonance effect that occurs when at least two, or more noble metal nanoparticles are brought in proximity within a distance equal to at most two and half time their average diameter.

The terms "pharmacokinetics" (PK), "ADME" and "toxicology" (Tox) as used herein refer to the absorption, distribution, metabolism, excretion and toxicological effects of drugs in an animal including mammals, such as humans, that may affect their efficacy as a pharmacological agent. In particular, these terms refer to the capacity of a drug, drug candidate, lead compound or NCE to distribute evenly or unevenly into the body of such an animal or human during a given timeframe, to access cells or parts of the organism such as the brain, to be metabolized rapidly or not, to bind to serum or plasma proteins or specific metabolizing enzymes such as, but not limited to, members of the cytochrome family, with more or less affinity, to interact with biological or synthetic membranes or layers, or to form covalent adducts with molecules or derivatives thereof involved in the metabolism, such as glucuronides, cysteine or glutathione.

The terms "reference" and "control" are used interchangeably to refer to a known value or set of known values against which an observed value may be compared. As used herein, known means that the value represents an understood parameter, e.g., affinity, percentage binding, volume of distribution, light absorption, light emission, etc.

The term "assessing" and "evaluating" are used interchangeably to refer to any form of measurement, and includes determining if an element present in the medium interacts with the colloid or a conjugate thereof. The terms "determining," "measuring," "assessing," and "assaying" are used interchangeably and include both quantitative and qualitative determinations. Assessing may be relative or absolute. "Assessing the reactivity of " includes determining the level of interaction between a pair of ligands, as well as between a ligand and a nanoparticle or a conjugate thereof.

As used herein, the term "detecting" means to ascertain a signal, either qualitatively or quantitatively.

The term "binding" refers to two objects associating with each other to produce a stable composite structure. In certain embodiments, binding between two complementary nucleic acids may be referred to as specifically hybridizing. The terms "specifically hybridizing," "hybridizing specifically to" and "specific hybridization" and "selectively hybridize to," are used interchangeably and refer to the binding, duplexing, or hybridizing of a nucleic acid molecule preferentially to a particular nucleotide sequence under stringent conditions. In certain embodiments, binding between a ligand and a receptor may be referred to as specifically interacting with a given affinity or avidity. In other embodiments, binding of a ligand to a molecule may refer to the formation of a covalent bond between a reactive structure of the ligand and a specific group of the target molecule.

By "competitive" or "competition" is meant herein that in a given assay one ligand competes with another or a protein-conjugate thereof for binding a receptor or an enzyme.

The terms "affinity" and "avidity" refer to the strength with which a pair of binding ligands associate with each another.

The term "screening" refers to determining the presence of something of interest, e.g., an analyte, an occurrence, etc. As used herein, the term "determining" means to identify, i.e., establishing, ascertaining, evaluating or measuring, a value for a particular parameter of interest, e.g., a hybridization parameter. The determination of the value may be qualitative (e.g., presence or absence) or quantitative, where a quantitative determination may be either relative (i.e., a value whose units are relative to a control or a known value (i.e., reference value) or absolute (e.g., where a number of actual molecules is determined).

The term "sample" as used herein refers to a fluid composition, where in certain embodiments the fluid composition is an aqueous composition.

DETAILED DESCRIPTION.

Methods using nanoparticle colloids, such as colloidal conductive polymers, noble metal nanoparticles or metal/conductive polymer composite colloids as reagents for at least the prediction of, if not the determination of, in vitro, PK/ADME-Tox properties of NCEs, drug candidates or lead compounds are provided. .

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

As summarized above, the subject invention provides methods, and reagents for at least the in vitro prediction of, if not the determination of, PK/ADME-Tox properties of NCEs, drug candidates or lead compounds (which may be collectively referred to herein as "candidate compounds"). As such, methods of determining at least a predicted PK/ADME-Tox property of a candidate compound are provided. In further describing aspects of the invention and certain embodiments thereof, representative examples and photonic properties of the subject colloids used are reviewed first in detail, followed by a discussion of representative fabrication protocols and methods for using the subject colloids within the scope of the present invention. In addition, a review of kits and devices that include the subject colloids as reagents and find use in practicing methods of the invention is provided.

CONDUCTIVE POLYMERS, METAL AND METAL/CONDUCTIVE POLYMER COMPOSITE COLLOIDS

As summarized above, the present invention uses the photonic properties of conductive polymers, metal or metal/conductive polymer composite colloids, to report (in an in vitro homogeneous assay system) the interaction of a ligand with a binding partner, an interacting structure such as a membrane, or a charge or redox-sensitive nanoparticle, so as to infer one or several of its PK/ADME-Tox characteristics, i.e., to predict in vitro one or more PK/ADME properties of a candidate compound.

Colloids finding use in embodiments of the invention are stable. As used herein, the term stable refers to the ability of the particles of the colloid to remain in suspension in the carrier medium of the colloid, e.g., the particles do not precipitate out of suspension to any significant extent. Another aspect of colloids that find use in methods of the invention is that they report a change in their physical environment such as refractive index, electric charge or redox potential, by a significant change in their photonic properties. As used herein and as known by one of skill in the art, the term photonic property refers to the capacity of these nanomaterials to absorb or scatter ultra-violet or visible light, emit phosphorescence, fluorescence or luminescence, etc.

As the subject colloids may be made of conductive polymers, metals or composites thereof, they include either a conductive polymer component, a metal component or both a metal component and a conducting polymer component. The metal component of colloids may be, when present, a noble metal. Noble metals of interest include, but are not limited to: Group VIII metals of the Periodic Table including, but not limited to: platinum, iridium, palladium and the like, as well as gold, silver, etc.

The term "conductive polymer" means an electrically conductive polymeric material. In certain embodiments, conductive polymers are organic polymers, such as p- or π-conjugated organic polymers. For example, employed may be polypyrroles such as polypyrrole, poly(N-substituted pyrrole), poly(3-substituted pyrrole), and poly(3,4-disubstituted pyrrole); polythiophenes such as polythiophene, poly(3-substituted thiophene), poly(3,4-disubstituted thiophene), and polybenzothiophene; polyisothianaphthenes such as polyisothianaphthene; polythienylenevinylenes such as polythienylenevinylene; poly(p-phenylenevinylenes) such as poly(p-phenylenevinylene); polyanilines such as polyaniline, poly(N-substituted aniline), poly(3-substituted aniline), and poly(2,3-substituted aniline); polyacetylnenes such as polyacetylene; polydiacetylens such as polydiacetylene; polyazulenes such as polyazulene; polypyrenes such as polypyrene; polycarbazoles such as polycarbazole and poly(N-substituted carbazole), polyselenophenes such as polyselenophene; polyfurans such as polyfuran and polybenzofuran; poly(p-phenylens) such as poly(p-phenylene); polyindoles such as polyindole; polypyridazines such as polypyridazine; polyacenes such as naphthacene, pentacene, hexacene, heptacene, dibenzopentacene, tertabenzopentacene, pyrene, dibenzopyrene, chrysene, perylene, coronene, Terylene, ovalene, quoterylene, and circumanthracene; derivatives (such as triphenodioxazine, triphenodithiazine, hexacene-6,15-quinone) which are prepared by substituting some of carbon atoms of polyacens with atoms such as N, S, and O, or a functional group such as a carbonyl group; polymers such as polyvinylcarbazoles, polyphenylenesulfide, and polyvinylenesulfide. Of particular interest in representative embodiments are polypyrolle, polythiophene, polyaniline or their derivatives.

In the composite colloids, metal particles are surface coated with a conducting polymer and suspended in a liquid medium, including but not limited to an aqueous medium, as described in published United States Patent application No. 20070172834 and published International application no. WO2006047371 (Englebienne and Van Hoonacker, 2006) . By "surface coated" is meant that at least a portion of the surface of the particles, if not the entire surface of the particles, is covered with a layer of conducting polymer molecules.

The dimensions of the particles may vary, but in certain embodiments range from about 1 nm to about 1 micrometer, such as from about 1 nm to about 100 nm, including from about 30 nm to about 60 nm. In certain embodiments, the particles have a narrow particle size distribution. By narrow particle size distribution is meant that the relative standard deviation of the particles is 30% or less, and in certain embodiments is 20% or less, e.g., is 17% or less, including 10% or less of the average diameter.

In certain embodiments, the particles are used either as a colloidal solution, or as a sensing film cast on a solid substrate, such as glass or other suitable solid material.

In certain embodiments, the particles display a receptor or a hydrophilic, hydrophobic or mixed membrane, e.g., that specifically or nonspecifically binds to a ligand of interest, on their surface. By display is meant that the receptor or the membrane is immobilized on the surface of the particle, where the receptor or membrane may be covalently or non-covalently bound to the surface of the particle. The density of the receptor molecules or of molecules making the membrane on the particle surface may vary, but may range from about 2 to about 50, such as from about 5 to about 25 molecules per particle.

As indicated above, a variety of different types of receptors or membrane layers may be displayed on the surface of the particles of the colloids. In certain embodiments, the particular receptor that is present depends on the PK/ADME-Tox property that is to be at least predicted, or determined, in a given application, such as the applications discussed below. Representative receptors of interest include, but are not limited to, the receptors discussed above, such as plasma binding proteins, enzymes of the cytochrome family, hydrophilic or hydrophobic membranes, detergents, nucleic acids, peptides, etc.

The pH of the colloid may vary, and in certain embodiments ranges from about 2 to about 12, such as from about 4.5 to about 9.0. The colloids may include a number of different additional components apart from the polymer coated metal particles, where additional components of interest include, but are not limited to: salts, buffering agents, detergents, stabilizers and the like.

METHODS OF FABRICATION

The subject colloids may be prepared using any convenient protocol that results in the production of a colloid of the invention, e.g., as described above. In one embodiment, a colloidal conductive polymer is produced by oxidation in aqueous acid of a suitable monomer in the presence of a stabilizing agent, such as a polymer as poly(vinylpyrrolidone) or poly(vinylalcohol). The colloidal conductive polymer may be purified by chromatography before further derivatization. In another embodiment, a noble metal colloid is prepared by reduction of a metal salt in water at a suitable temperature.

Respective examples of protocols for the production of such colloidal nanoparticles are provided in the book Immune and Receptor Assays in Theory and Practice (Englebienne, P. (2000) Immune and Receptor Assays in Theory and Practice. CRC Press, Boca Raton, 392pp) on pages 243 and 234, respectively. In another embodiment, an initial or precursor metal colloid and a water-soluble conductive polymer are combined with each other in a manner sufficient for the water-soluble conductive polymer to adsorb to the surface of particles of the metal colloid and thereby produce the product composite colloid used in the invention. Protocols for the production of such stable composite nanocolloids are provided in published United States Patent application No. 20070172834 and published International application no. WO2006047371 (Englebienne and Van Hoonacker, 2006) .

In some embodiments pertaining to the present invention, the colloids further display an agent that interacts with the candidate compound of interest, where the agent may be a variety of entities (as indicated above), including but not limited to: receptors, enzymes, natural or synthetic membranes, nucleotides, antibodies, ligands, or derivatives thereof, e.g., for biosensing purposes. These displayed molecules may be attached to the particle surface by charge adsorption or by covalent binding, as convenient. Any suitable coupling protocols may be employed.

UTILITY

The subject colloids, as described above, may be employed in a variety of different applications intended at predicting or deducing the PK/ADME-Tox characteristics of a candidate compound, e.g., NCEs, drug candidates or lead compounds. The methods may be used to at least predict, if not determine, one or more different PK/ADME-tox properties of a candidate compound.

In certain embodiments, the property of interest is absorption. Before a compound can exert a pharmacological effect in tissues, it has to be taken in to the bloodstream - usually via mucous surfaces like the digestive tract (intestinal absorption). Uptake into the target organs or cells needs to be ensured, too. This can be a serious problem at some natural barriers like the blood-brain barrier. Factors such as poor compound solubility, chemical instability in the stomach, and inability to permeate the intestinal wall can all reduce the extent to which a drug is absorbed after oral administration. Absorption critically determines the compound's bioavailability. Drugs that absorb poorly when taken orally must be administered in some less desirable way, like intravenously or by inhalation.

In certain embodiments, the property of interest is distribution. The compound needs to be carried to its effector site, most often via the bloodstream. From there, the compound may distribute into tissues and organs, usually to differing extents.

In certain embodiments, the property is metabolism. Compounds begin to be broken down as soon as they enter the body. The majority of small-molecule drug metabolism is carried out in the liver by redox enzymes, termed cytochrome P450 enzymes. As metabolism occurs, the initial (parent) compound is converted to new compounds called metabolites. When metabolites are pharmacologically inert, metabolism deactivates the administered dose of parent drug and this usually reduces the effects on the body. Metabolites may also be pharmacologically active, sometimes more so than the parent drug.

In certain embodiments, the property is excretion. Compounds and their metabolites need to be removed from the body via excretion, usually through the kidneys (urine) or in the feces. Unless excretion is complete, accumulation of foreign substances can adversely affect normal metabolism.

In certain embodiments, the property of interest is $pK_a$. The $pK_a$ value(s) of a compound influence many characteristics of the compound such as its reactivity, and spectral properties (colour). In biochemistry the $pK_a$ values of proteins and amino acid side chains are of major importance for the activity of enzymes and the stability of proteins. This property is of general importance in chemistry because ionization of a compound alters its physical behavior and macro properties such as solubility and lipophilicity. For example, ionization of any compound will increase the aqueous solubility, but decrease the lipophilicity. This can be exploited in drug discovery to increase the concentration of a compound in the blood by adjusting the $pK_a$ of an ionizable group at a relevant pH, this must be done with caution, however, since an ionized compound will be less permeable through cell membranes.

In certain embodiments, the property is toxicity. Toxicity is a measure of the degree to which something is toxic or poisonous. Toxicity can refer to the effect on a whole organism, such as a human, or to a substructure, such as a cell (cytotoxicity) or an organ (organotoxicity such as the liver (hepatotoxicity).

In certain embodiments pertaining to the present invention, the subject colloids may be used to screen a sample containing such a ligand for binding to a plasma protein, interaction with a natural or synthetic membrane, or an enzyme such as a member of the cytochrome P450 family, a cell membrane channel such as a member of the ATP-binding cassette transporters such as the multidrug resistance protein-1, etc., where these receptors are displayed on the nanoparticles. In other embodiments, nanoparticles stabilized with a detergent at a suitable pH may be contacted with the sample containing the ligand so as to determine its electrical charge or redox potential. The nanoparticles may also display molecules on their surface with which the ligand may form a covalent adduct. In some embodiments, the ligand of interest may enter in competition with another ligand for binding a receptor, an enzyme, etc.

In such applications, a volume of colloid or a fixed colloid quantity cast on a solid support, e.g., a colloid that is naked or includes a receptor or an enzyme specific for the ligand, a natural or synthetic membrane respectively, is contacted with the sample to be screened, and an optical parameter of the colloid is monitored to detect a change therein upon interaction, e.g., a change in absorption of the colloid at a given wavelength. Any convenient optical parameter may be assessed or monitored in this step, where representative parameters include, but are not limited to: absorption, scattering, fluorescence, luminescence and the like. The optical parameter may be monitored using any convenient device and protocol, where suitable protocols are well known to those skilled in the art and representative protocols are described in greater detail in the experimental section, below.

The presence or absence of a change in the optical parameter is then used to make a determination of whether or not the ligand of interest interacts with a receptor, competes with another ligand for a receptor, or the nanoparticle and to which extent.

By referencing to a control, e.g., data obtained from the same system using a control known drug that has been extensively studied by pharmaceutical scientists and of which PK/ADME-Tox characteristics have been published in the literature, the results of the assays are related to specific PK/ADME-Tox characteristics such as the logP, logD, charge, redox potential, volume of distribution, cell or blood brain barrier penetration, metabolic transformation and possible toxicity, etc. In certain embodiments, a calculated parameter, e.g. the affinity of the ligand for a receptor, can be obtained from the optical result and one may calculate directly the PK/ADME-Tox parameter from any convenient equation, such as those currently known to one of skill in the art.

The $pK_a$ of a compound allows one to calculate its percentage ionized at a given pH (e.g., in different body organs such as blood, duodenum, ileum, etc.) according to the following relationship:

$$\%ionized = 100\frac{1}{1 + 10^{\pm(pH - pKa)}}$$

where the exponent sign is + for bases and - for acids.

Similarly, the log distribution coefficient (D) of ionized species of a molecule, which at pH 7.4 is directly correlated to clearance ($C_L$), can be calculated from the octanol/water partition coefficient ($Po/w$) and the $pK_a$ according to Scherrer's relationship:

$$LogD(pH) = \log Po/w - \log[1 + 10^{(pH - pKa)}]$$

for monoprotic acids, although for monoprotic bases the exponent is written as $pK_a$ - pH. In more complex situations, where a polyprotic molecule is involved, the second term of the above equation becomes:

$$\log[1 + 10^{-(\sum_1^n pKa - npH)} + 10^{-(pKa_1 - pH)}]$$

When a nanoparticle is surrounded by a membrane or a cell layer, the signal resulting from the interaction of a molecule with the nanoparticle, through the layer during time, is directly related to the transfer rate of the drug (dQ/dt) and allows therefore to calculate an apparent permeability coefficient ($P_{app}$) through tissues:

$$P_{app} = \frac{dQ}{dt}\frac{1}{AC_0}$$

where A is the layer surface area and $C_0$ the initial concentration. This relationship further allows to approximating the fraction of drug unbound in tissues ($fu_T$). Similarly, the affinity constants ($K_A$) which govern the binding of a molecule to the n independent binding sites of serum proteins such as albumin and $\alpha_1$-acid glycoprotein allows one to calculate the fraction of drug unbound in plasma ($fu_P$):

$$fu_P = 1 - \left[\frac{nK_A}{1+K_A}\right]$$

In turn, the Oie-Tozer equation further allows one to infer an idea of the volume of distribution at steady state ($V_{Dss}$):

$$V_{Dss} = V_P\left(1 + R_{E/i}\right) + fu_P V_P\left(\frac{V_E}{V_P} - R_{E/i}\right) + \frac{V_R fu_P}{fu_T}$$

with $V_P$ and $V_E$ the plasma and extracellular fluid volumes (0.0436 and 0.151 UKg body weight respectively), $R_{E/i}$ is the ratio of extravascular to intravascular proteins (taken as 1.4) and $V_R$ is the physical volume into which the drug is distributed minus the extracellular space (0.380 UKg body weight).

The interaction isotherm of a molecule with the cytochrome P450 isozymes provides an approximation of the $V_{max}/K_m$ ratio corresponding to its intrinsic clearance ($CL_{int}$), which further allows one to approximate the total clearance ($CL_{tot}$) which equals the product of $CL_{int}$ by $fu_T$. These complementary relationships finally permit one to deduce some pharmacokinetic parameters, such as the half-life after oral administration ($t_{1/2}$):

$$t_{1/2} = \frac{0.693 V_{Dss}}{CL_{tot}}$$

In the broadest sense, the methods may be qualitative or quantitative. As such, where detection is qualitative, the methods provide a reading or evaluation, e.g., assessment, of whether or not the target ligand present in the sample being assayed interacts or not with the nanoparticles. In yet other embodiments, the methods provide a quantitative detection of whether the target ligand present in the sample being assayed interacts progressively in increasing concentrations with the colloids, i.e., an evaluation or assessment of the actual amount of the target ligand in the sample being assayed that interacts with a fixed quantity of the colloid. In such embodiments, the quantitative detection may be absolute or, if the parameter studied is known or has been assessed by other methods, relative. As such, the term "quantifying" when used in the context of quantifying a parameter such as affinity of target ligand(s) in a sample can refer to absolute or to relative quantification. Absolute quantification may be accomplished by inclusion of known concentration(s) of the ligand in the assay and analyzing the binding isotherm. Alternatively, relative quantification can be accomplished by comparison of optical signals generated by the target ligand(s) and by reference ligand(s), all interacting with the same colloid, to provide a relative quantification of each of the two or more different ligands, e.g., relative to each other.

The subject methods can be employed to detect the interaction of one or more target ligands with the colloids in a variety of different types of samples, including complex samples including organic solvents miscible with an aqueous solution, such as dimethyl sulfoxide, dimethylformamide, ethanol or methanol and the like, where the subject methods provide for detection of the target ligand(s) interaction with high sensitivity.

The methods of the present invention may be used to detect the interaction of a wide variety of ligands with the nanoparticles. Ligands of interest can be an organic or inorganic molecule, including inorganic or organic salts, proteinacious molecules, such as, but not limited to, proteinacious ligands, including peptides and proteins and fragments thereof, as well as nucleic acids and other types of ligands, where the ligands may be a single molecule, a complex that includes two or more molecular subunits, which may or may not be covalently bound to each other, a microorganism, e.g., virus or single celled pathogen, a cell, a multicellular organism or portion thereof, and the like.

In addition, the subject methods may also be used to screen for compounds that modulate the interaction of a given specific binding member pair. The term modulating includes both decreasing (e.g., inhibiting) and enhancing the interaction between the two molecules. For example, where the colloid displays a first member of a binding pair and the colloid is contacted with the second member in the presence of a candidate agent, the effect of the candidate agent on the interaction of the binding member pairs can be evaluated or assessed.

A variety of different ligands and candidate agents may be screened by the above methods. These ligands and candidate agents encompass numerous chemical classes, including but not limited to organic molecules, such as small organic compounds having a molecular weight of more than 50 and less than about 2,500 daltons. Ligands and candidate agents comprise functional groups necessary for structural interaction with proteins or other receptors displayed by the nanoparticles, particularly hydrogen or hydrophobic bonding, and may include at least an amine, carbonyl, acyl, an halogen, hydroxyl or carboxyl group, such as at least two of the functional chemical groups. The ligands and candidate agents often comprise cyclical carbon or heterocyclic structures and/or aromatic or polyaromatic structures substituted with one or more of the above functional groups. Ligands and candidate agents are also found among biomolecules including peptides, saccharides, fatty acids, steroids, purines, pyrimidines, derivatives, structural analogs or combinations thereof.

Ligands and candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

In certain embodiments, the subject methods are performed in a high throughput (HT) format. In the subject HT embodiments of the subject invention, a plurality of different compounds are simultaneously tested. By simultaneously tested is meant that each of the compounds in the plurality are tested at substantially the same time. Thus, at least some, if not all, of the compounds in the plurality are assayed for their effects in parallel. The number of compounds in the plurality that are simultaneously tested may be 5 or more, such as 10 or more, where in certain embodiments the number may be 100 or more or 1000 or more, where the number of compounds tested may be higher. The number of compounds that are tested simultaneously in HT embodiments may range from 10 to 10,000, such as from 100 to 10,000 and including from 1000 to 5000. A device may have as many containers as the compounds to be tested, e.g., 5 or more, 10 or more, 100 or more, 1000 or more, 5000 or more, 10,000 or more, etc. Of interest in certain embodiments are 96 well plates, 384 well plates 1536 well plates, etc. A variety of high throughput screening assays for determining the activity of candidate agent are known in the art and are readily adapted to the present invention, including those described in issued U.S. Patent No. 6,127,133 .

Agents identified in the above screening assays find use in a variety of methods, including methods of modulating the activity of the target ligand on a given receptor, and conditions related to the activity thereof.

The subject methods can be used to predict or determine a candidate compound's, e.g. NCEs, drug candidates or lead compounds, viability as a potentially marketable therapeutic agent. As discussed above, acceptable pK/ADME-Tox characteristics may facilitate a candidate compound's therapeutic effectiveness. To determine whether a candidate compound has acceptable pK/ADME-Tox characteristics, methods of the subject invention may be performed to obtain results corresponding to properties of interest, such as but not limited to $pK_a$, absorption, distribution, metabolism, excretion and toxicity. These results may be compared to a control value. In certain embodiments, control value is a value obtained using an assay according to the invention for a known compound of known pk/ADME-tox properties. In some embodiments, the control value may be equal to or substantially the same as the corresponding value for a known chemical compound that has acceptable pk/ADME-Tox characteristics. In some embodiments, the control value may be equal to or substantially the same as the average of the corresponding values for two or more known chemical compounds that have acceptable pk/ADME-Tox characteristics. As used herein, the terms "substantially the same" or "substantially similar" refer to a value that is equivalent to another value within a specified range of values, for example ±40%, including ±20%, such as ±10% or ±5%.

In some cases, the results obtained from the subject method for a candidate compound may be compared to the control value for a property of interest, as described above. If the result obtained from the subject method is substantially the same, i.e., within a certain range of values, for example ±40%, including ±20%, such as ±10% or ±5%, as the control value, then the candidate compound has an acceptable value for the property of interest. If the candidate compound has an acceptable value for the property of interest, then it may be more likely that the candidate compound is viable as a potentially marketable therapeutic agent. The above comparisons of results obtained from the subject methods with control values may be performed for one or more properties of interest. Acceptable values for one or more properties of interest indicate that the candidate compound may be suitable for further testing, such as but not limited to further *in vitro* studies, *in vivo* studies in animals, or human clinical trials. As such, certain embodiments further include determining whether a candidate compound has an acceptable value and, if so, performing one or more subsequent test, e.g., further *in vitro* studies, *in vivo* studies in animals, or human clinical trials.

Conversely, if the result obtained from the subject method is not substantially the same as the control value, then the candidate compound does not have an acceptable value for the property of interest. If the candidate compound does not have an acceptable value for the property of interest, then this may indicate that the candidate compound is less likely to be viable as a potentially marketable therapeutic agent. Unacceptable values for one or more properties of interest indicate that the candidate compound may not be suitable for further testing. A decision may then be made not to test the compound further.

The following examples are offered by way of illustration and not by way of limitation.

EXPERIMENTAL

EXAMPLE 1.

A solution of gold nanoparticles of an average diameter of 40 nm was prepared by dissolving in a 1 liter Erlenmeyer 200mg of Hydrogen tetrachloroaurate (III) (Acros 22362-0010) in 500 ml of distilled water. A magnetic bar was added and the vessel was placed on a magnetic stirrer. The solution was heated to a boil and agitated vigorously. When boiling, 30ml of a 1% sodium citrate (prepared by neutralizing a 1% citric acid - anhydrous, Sigma C-0759) were injected rapidly into the vessel. Boiling and stirring were continued until the solution turned progressively black and then deep red, indicating the formation of the nanoparticles. The solution was then cooled to room temperature. The sol was characterized by an absorption band at 524 nm and an optical density (1cm) of 4.

A 10ml portion of the colloidal gold solution was then poured in a beaker with a magnetic stirring bar and was progressively neutralized under magnetic stirring up to pH 6.5 using 10 mM sodium hydroxide. An aqueous solution of bovine serum albumin (defatted BSA, Equitech Inc., 750 mg/ml, 2ml) was rapidly added under magnetic stirring. The mixture was allowed to react during 10 minutes at room temperature and was then buffered by the addition of 228 mg of sodium tetraborate decahydrate (Fluka 72000). The pH was adjusted to $9\pm0.1$ and the solution was stored in a brown glass bottle and used as the colloid displaying the albumin receptor on its surface. BSA is often substituted to human serum albumin in pharmaceutical research because both proteins display a very similar behavior toward drugs.

Solutions (100 mM) of theophylline (Fluka 88308) and bilirubin (Aldrich R750972) were prepared in dimethylsulfoxide (DMSO, Fluka 41640). These solutions were then diluted 100 fold in borate buffer pH 9 (50 mM, containing 10 mM magnesium sulfate) so as to generate 1 mM buffered solutions containing 1% DMSO. These solutions were then serially diluted in the same borate buffer containing 1% DMSO to generate lower concentration ligand samples.

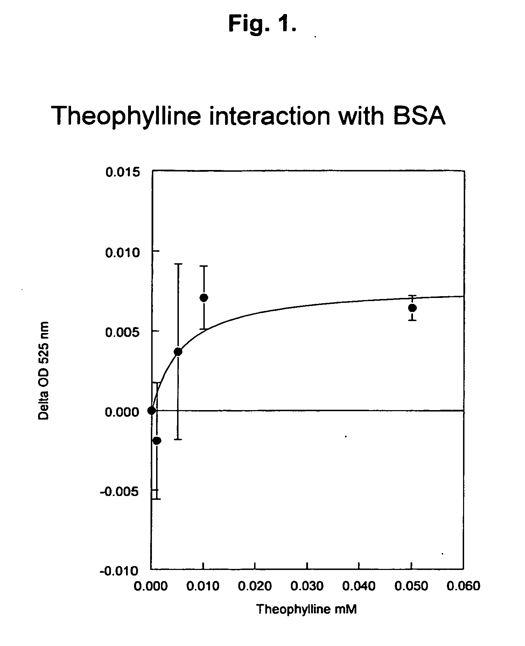

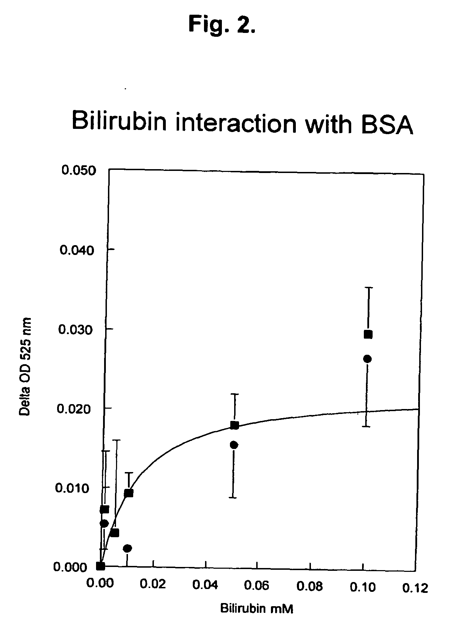

These ligand solutions (30 $\mu$l) as well as a blank solution (buffer with 1% DMSO devoid of ligand) were then pipeted in duplicate in a 96 well plate. Borate buffer (120 $\mu$l of 50mM pH 9, containing 10mM magnesium sulfate and 40g/l poly(ethylene glycol) 6,000 - Fluka 81260) was then added along with 50$\mu$l of the BSA-gold colloid conjugate. The plate content was thoroughly mixed and incubated during 4 hours at room temperature. The optical density of individual wells at 525 nm was then read in a Spectramax Plus plate reader (Molecular Devices). The O.D. of the blank solution was subtracted from each result. The respective O.Ds. were plotted versus the ligand concentrations so as to generate the binding isotherms shown respectively in Fig. 1 (theophylline) and Fig. 2 (bilirubin). In each Figure, the O.D. signal is shown as the average $\pm$ s.d. of duplicates. Fig. 2 further shows the results of two separate experiments.

The binding isotherm shown in Fig. 1 allows one to estimate the affinity of theophyllin for BSA as being $5.1.10^4$ liter per mole with an $r^2$ of 0.82. Similarly, the binding isotherm displayed in Fig. 2 allows one to estimate the affinity of bilirubin for BSA as being $1.2.10^8$ liter per mole with an $r^2$ equal to 0.73. These two ligand examples were selected because of their differential binding affinity for albumin. Bilirubin is the single most well-studied ligand of albumin which it is estimated to bind with an averaged affinity of $9.5.10^7$ liter per mole (Peeters, T.Jr. (1996) All about Albumin. Academic Press, San Diego, 432pp p.97). At the opposite, theophylline is bound by albumin with a much lower affinity, which makes the former to be close to 100% bound by serum albumin although $56\pm4\%$ only of the latter is bound by the protein in plasma (Goodman and Gillman (1996) The Pharmacological Basis of Therapeutics, 9th ed. (Hardman, J.G. et al. eds). McGraw-Hill, New York). From the affinity of theophylline obtained by the present technique, it is possible to deduce the fraction bound in plasma (0.558 which is very close to the average of 0.56 obtained experimentally as given above) and hence its volume of distribution by using published equations (Lombardo, F., Shalaeva, M.Y. et al. (2000) ElogPoct: A tool for lipophilicity determination in drug discovery. J. Med. Chem. 43, 2922-2928). By introducing these data into such equations, a volume of distribution for theophylline in humans of 0.551 liters per Kg is obtained, which falls within the range of $0.50\pm0.16$ liters per Kg observed experimentally (Goodman and Gillman, 1996, supra). The volume of distribution of a drug is an important parameter that further allows to calculate the area under curve (AUC) of the pharmacokinetics for a given dose.

EXAMPLE 2.

Colloidal poly(aniline) was obtained as follows: As much as possible of a 10g portion of Poly(vinyl alcohol) was dissolved in 200 ml boiling water. The hot solution was then filtered and cooled to room temperature. The solution was then acidified by adding 16.8 ml of HCl 12N. A portion of this solution (100 ml) was transfered to a clean beaker with a magnetic bar, which was placed on a magnetic stirrer at 4°C. The solution was agitated and 0.25 ml of aniline (Aldrich 24,861-4) was injected. Immediately after this injection, 658 mg of ammonium peroxidisulfate (Aldrich 24,861-4) freshly dissolved in 5 ml water were added. The solution was maintained under stirring for 48 hours at 4°C. The colloid was then purified by chromatography on a Sephacryl S-1000 column eluted with 0.1 M phosphate buffer pH 8 containing 0.5% of tween 20.

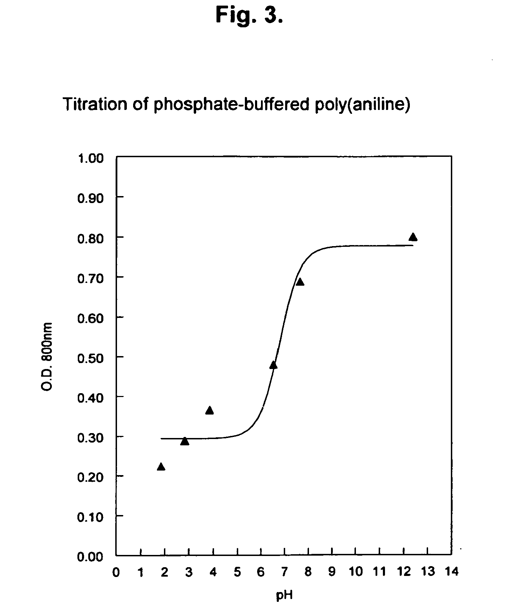

The buffered solution was then titrated with either HCl or NaOH and a spectrum was taken at each pH value recorded during the titration (Ultrospec 2000, Pharmacia). The absorbance at the peak corresponding to the progressive formation of the emeraldine base (800 nm) within this pH range allows to identify the pKa of the phosphate anion (theoretical: 7.21; calculated from the curve: 6.80). This is exemplified in the Fig. 3.

Thus, as shown by this example, the assay system can be used to measure the pKa of molecules, including candidate drugs, a parameter which is critical as it has a pronounced effect on pharmacokinetics (absorption and membrane permeation).

EXAMPLE 3.

A colloidal gold sol was prepared as described above in example 1. The colloid was then coated with human serum albumin (HSA, Sigma A-1653) as described above, except that the gold colloid was set at pH 5.5. The reagent was then buffered with 50mM borate as above and 150mM sodium chloride, Tween 20 (0.2%) and Fish gelatin (0.1%) were respectively added for further stabilization.

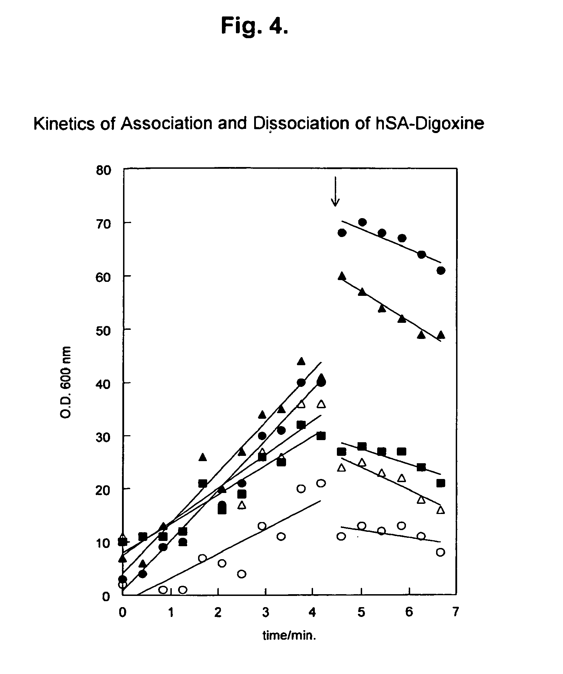

Digoxin (Fluka 37100) was dissolved in methanol (100 mg/liter, 128$\mu$M) and further diluted in PBS to generate sample solutions containing respectively 256, 128, 64, 25.8 and 12.8 nM. These samples (30 $\mu$l) were mixed with an accelerating buffer (120$\mu$l; 200 mM Tris-HCl pH 8 containing 250 mM magnesium chloride, 70g/l poly(ethyleneglycol) 6000, 15g/l poly(ethyleneglycol) 35,000 and Triton X-100 10ml/l). The mixing and reaction was performed in disposable cuvettes in a clinical chemistry analyzer thermostated at 37°C (Cobas Mira Plus, ABX Diagnostics, Montpellier, France). Immediately after sample and buffer mixing, the gold-HSA conjugate (50$\mu$l) was added to the reaction mixture. The association kinetics was followed at 600 nm and after a few minutes, a 500 fold excess of digoxin (20 $\mu$l) was injected in the reaction mixture and the dissociation was further followed at the same wavelength in the automated instrument. The results are displayed in Fig. 4 and the arrow indicates the time of ligand excess addition. Linear regressions calculated for both association and dissociation curves provided with coefficients of correlation between 0.86 and 0.99.

The analysis of the association and dissociation kinetics allowed the calculation of an average affinity for the drug-HSA interaction of $1.6\pm2.5.10^5$ liters per mole (mean $\pm$ s.d. of five assays). The apparent affinity of digoxin for human serum albumin determined by dialysis is $1.85.10^5$ liters per mole (Westphal, U. (1986) Steroid-Protein Interactions II. Springer-Verlag, Berlin, 603pp).

EXAMPLE 4.

A composite colloid made of gold nanoparticles and poly(o-anthranilic acid) was prepared as extensively described in published United States Patent application No. 20070172834 and published International application no. WO2006047371 (Englebienne and Van Hoonacker, 2006) .

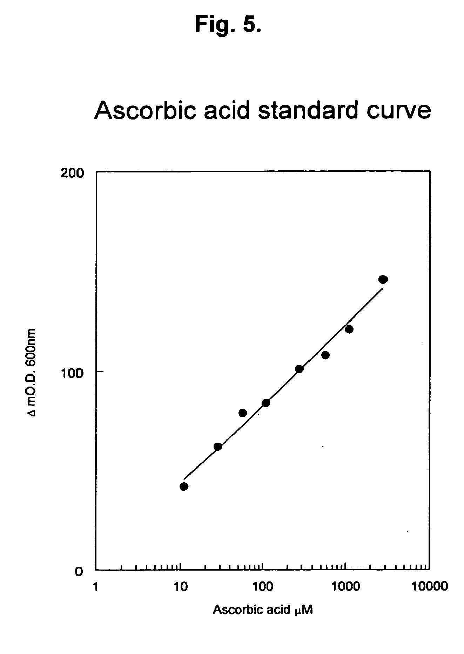

The composite solution was buffered at pH 5 by addition of 50 mM of sodium acetate. The composite solution (0.5 ml) was then reacted during 10 minutes at room temperature with an equal volume of aqueous solutions made in 3% acetic acid, and containing increasing concentrations of ascorbic acid. A blank was prepared by mixing the same volumes of the colloid and 3% acetic acid in water. After incubation, the change in optical density at 600 nm for the ascorbic acid samples versus the blank was recorded and plotted versus the ascorbic acid concentrations so as to generate a standard curve. The results are displayed in Fig. 5. Ascorbic acid is a strong reductant and a similar, but opposite curve can be generated with a strong oxidant because of the exquisite sensitivity of the colloids to oxido-reduction. Consequently, the redox potential of a given drug candidate can be evaluated rapidly by allowing its reaction at a given pH and concentration with the colloids, and by comparing its optical reactivity to that of known oxidants or reductants such as in the example shown on Fig. 5. As an example, the molecule Trolox® (6-Hydroxy-2,5,7,8-tetramethylchroman-2-carboxylic acid), a water-soluble Vitamin E derivative, displays a redox potential similar to ascorbic when tested in the same conditions, which is in full accordance with published data.

As further described in International publication no. WO03027656 , the redox potential of an NCE, drug candidate or lead is an important parameter which might lead to decide whether or not the molecule is developed further. Indeed, although a reductant drug may exert beneficial therapeutic effects, an oxidant may be toxic (Shargel, L. and Yu, A.B.C. (1999) Applied Biopharmaceutics and Pharmacokinetics, 4th Ed. McGraw-Hill, New York, 768pp). The redox potential level of a drug further governs also the kind and rapidity of its metabolism and henceforth modulates its pharmacokinetic profile.

EXAMPLE 5.

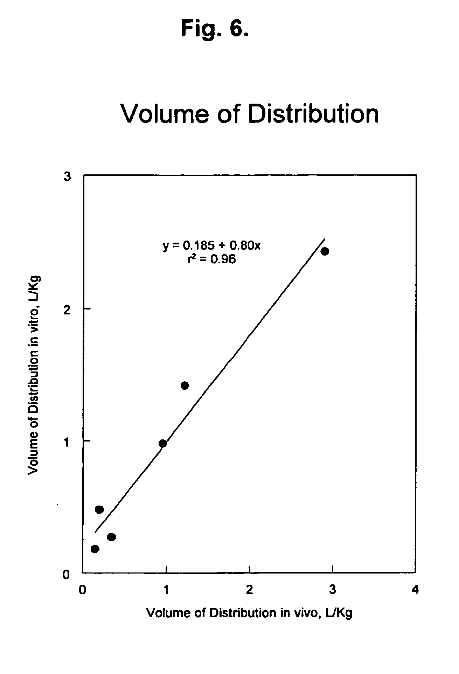

The affinity for bovine serum albumin of a series of known drugs was assayed according to the procedure given in Example 1. These drugs are either poor binders (Paracetamol and Acetylsalicylic acid), medium binders (Erythromycin, Theophyllin, Chlorpheniramine), or a high binder (Salicylate) to the protein. The log of the affinity constant calculated for each binding pair was then plotted on a graph versus the percentage of binding to the plasma proteins in vivo as obtained from published data (Goodman and Gillman, 1996, supra). The non-linear least-square regression provided a squared correlation coefficient of sufficient importance ($r^2 = 0.95$) as to allow for the deduction, with confidence, of the plasma protein binding data in vivo of a given sample from the affinity for albumin as obtained in vitro. The fraction of plasma protein-bound drug further allows one to calculate its volume of distribution, which is an essential parameter for characterizing the pharmacokinetics of a drug. Fig. 6 shows the regression and correlation functions between the apparent volume of distribution obtained with data for the six drugs of this example (ordinate), with the volume of distribution obtained from in vivo pharmacokinetics (abscissa, Goodman and Gillman, 1996, supra).

## EXAMPLE 6.

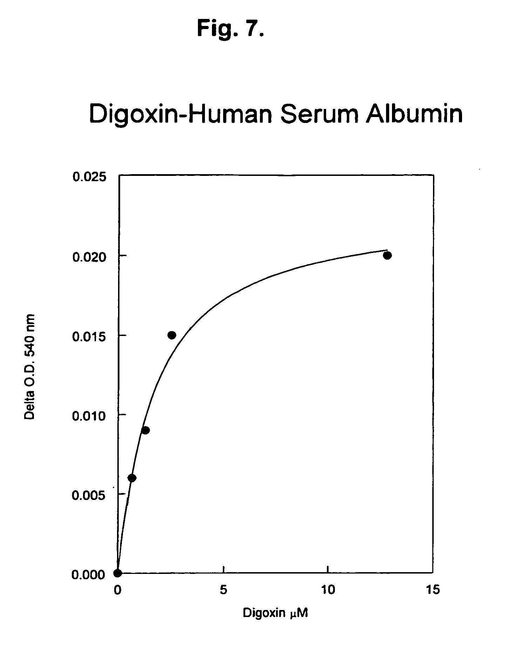

A gold colloid containing nanoparticles displaying human serum albumin (HSA, Sigma A-1653) at their surface was prepared as described in Example 3. Digoxin (Fluka 37100) was dissolved in methanol (100 mg/liter, 128μM) and further diluted in PBS to generate sample solutions containing respectively 12.8, 6.4, 2.5, and 1.25 μM. These samples and a PBS blank (100 μl) were placed in respectively labeled disposable plastic tubes to which the accelerating buffer (600μl; 200 mM Tris-HCl pH 8 containing 250 mM magnesium chloride, 70g/l poly(ethyleneglycol) 6000, 15g/l poly (ethyleneglycol) 35,000 and Triton X-100 10ml/l) and the gold-HSA conjugate (200μl) were added. After vortex-mixing, the tubes were incubated for 10 minutes at room temperature. After incubation, the optical density at 540 nm was recorded in a spectrophotometer (Ultrospec 2000, Pharmacia) zeroed on the blank. The increase in optical density of each sample was then plotted versus the concentration. The plot is shown in Fig. 7. From the binding isotherms calculated from two such independent experiments, an averaged apparent affinity of $7.5 \pm 2.3.10^5$ was obtained. The apparent affinity of digoxin for human serum albumin determined by dialysis is $1.85.10^5$ liters per mole (Westphal, 1986, supra).

## EXAMPLE 7.

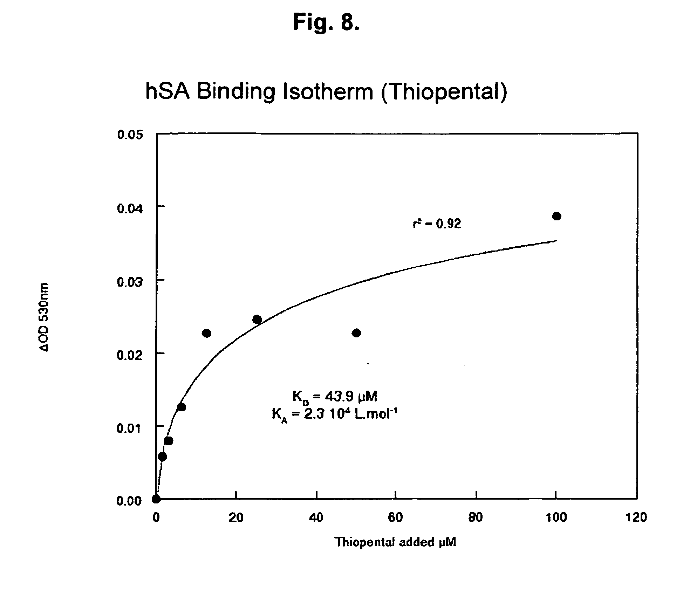

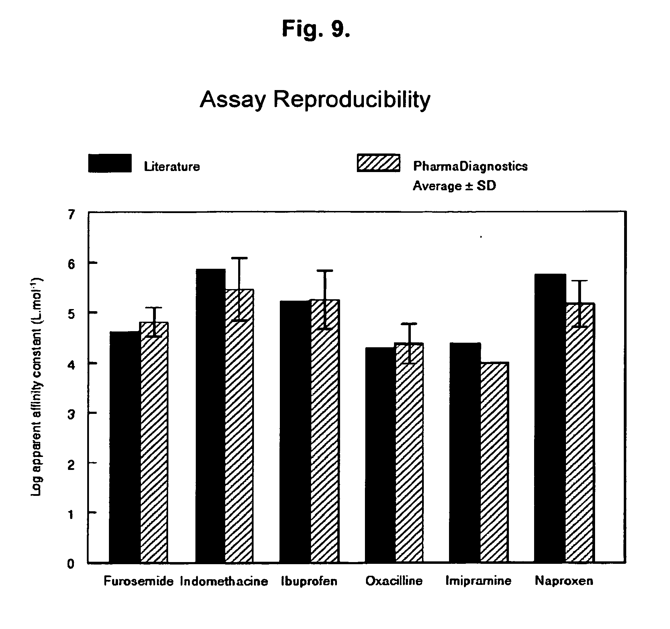

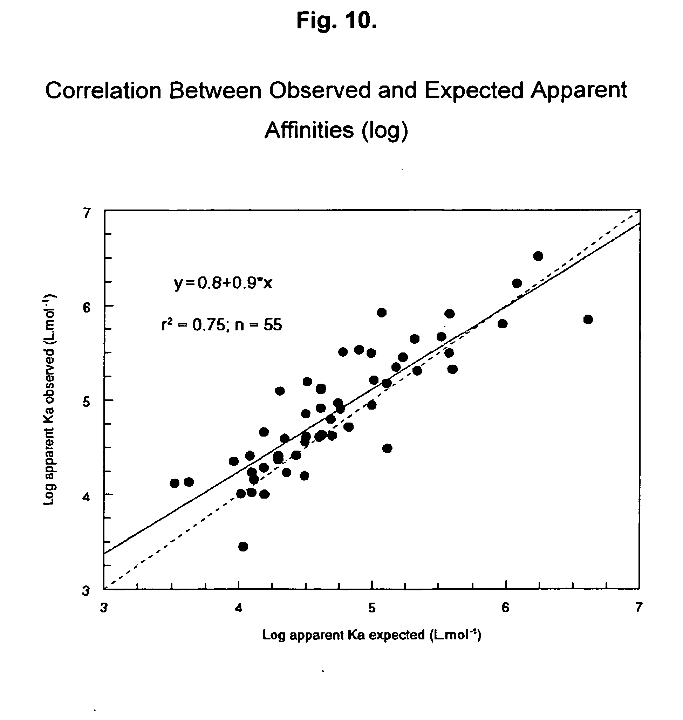

Serial dilutions of drugs were incubated in duplicate during 3 hours RT with colloidal gold nanoparticles bearing human serum albumin on their surface. The UV-vis signal resulting from the drug-protein interaction was plotted vs concentration added and the apparent affinity constant was calculated from the binding isotherm. Fig. 8 shows an example of such a binding isotherm. Fig. 9 shows the assay reproducibility for six known drugs: furosemide, indomethacine, ibuprofen, oxacilline, imipramine, and naproxen. The drugs were randomly dispensed in three separate plates. Affinity data was compared to data from the literature. Fig. 10 shows the correlation between the apparent affinity observed with this technique and apparent affinity data from the literature (experimental or calculated from % serum protein binding). The dashed line indicates identity.

## EXAMPLE 8.

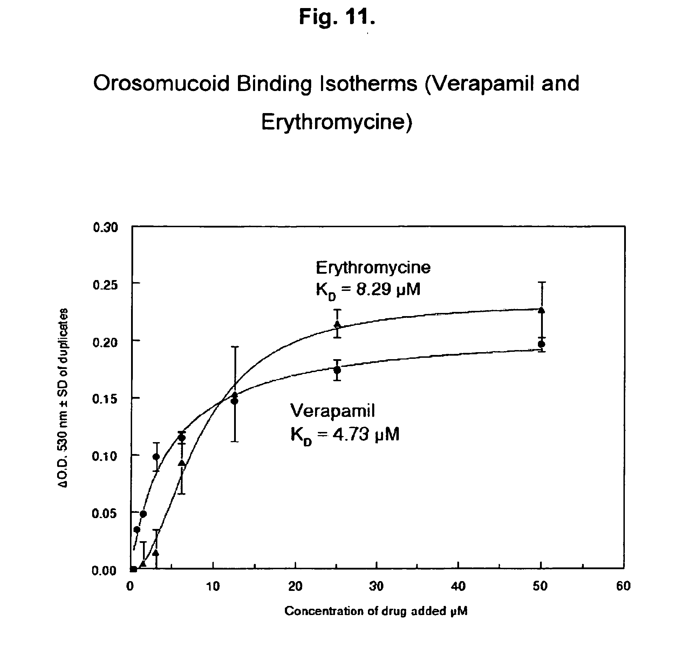

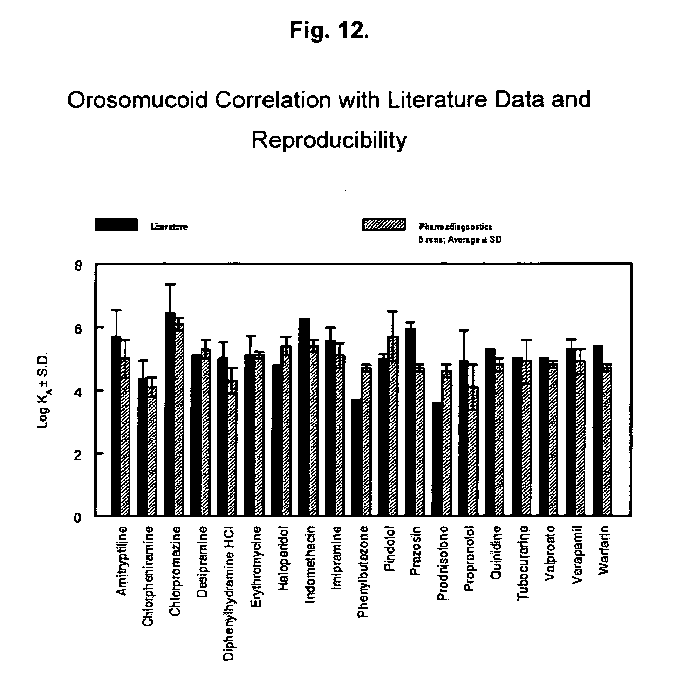

Serial dilutions of drugs were incubated in duplicate during 2 hours RT with colloidal gold nanoparticles bearing human orosomucoid (α1- acid glycoprotein) on their surface. The UV-vis signal resulting from the drug-protein interaction was plotted vs concentration added and the apparent affinity constant was calculated from the binding isotherm. Fig. 11 shows examples of such binding isotherms for verapamil and erythromycine. Fig. 12 shows the assay reproducibility and the correlation of the apparent affinities observed for 19 known drugs with those obtained from the literature. The 19 known drugs were amitryptiline, chlorpheniramine, chlorpromazine, desipramine, diphenylhydramine HCl, erythromycine, haloperidol, indomethacin, imipramine, phenylbutazone, pindolol, prazosin, prednisolone, propranolol, quinidine, tubocurarine, valproate, verapamil, and warfarin.

## EXAMPLE 9.

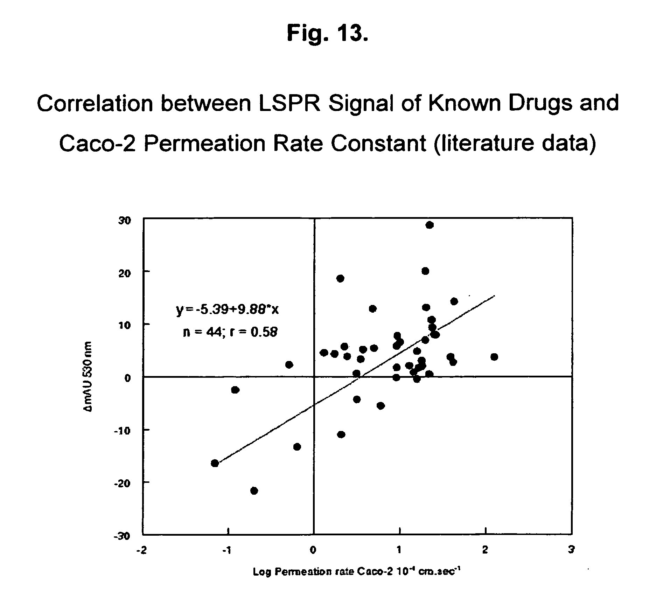

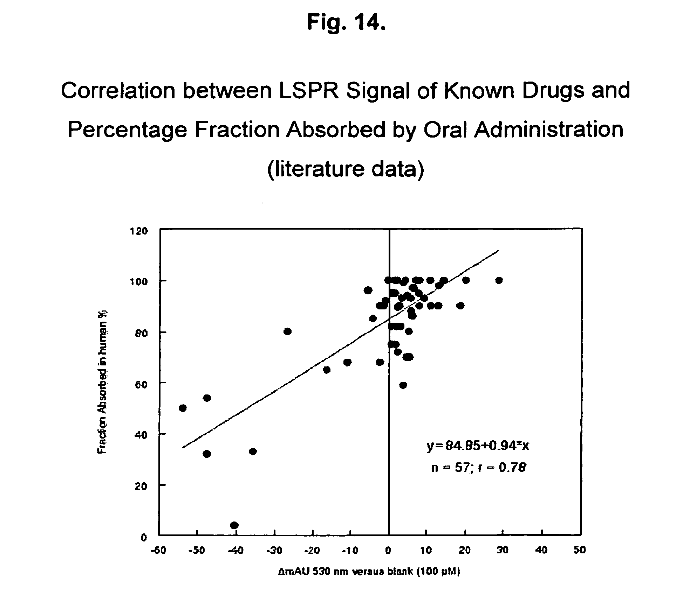

A solution of 40nm colloidal gold nanoparticles was prepared as described in Example 1. The colloidal nanoparticles so obtained (pH 4.76) were then coated with 11-mercaptoundecanoic acid (95%, Aldrich 450561) as follows: 100 mL of the colloidal gold solution was mixed with 0.4 mL of a 1:10 aqueous solution of Tween 20 (Aldrich 274238) and allowed to incubate under magnetic stirring for 20 minutes at RT. A 1mg/mL solution of 11-mercaptoundecanoic acid was prepared in ethanol. Two hundred microliters (0.2 ml; 200 μg) of this solution was diluted in 9.8 mL ethanol and mixed with the colloid under magnetic stirring. The mixture was then incubated for 2.5 hours at RT. This procedure provided nanoparticles fully stabilized with a membrane made of a hydrophobic alkane chain attached to the particles by the thiols and terminated at the outside by the carboxylic groups of the mercaptoundecanoic acid, thereby mimicking a physiological bilayer membrane. The final pH (5.09) of the solution approximated that in human duodenum. This nanoparticle solution was then diluted 1:4 in water and 0.198mL was pipeted in polystyrene 96 well plates using a Tecan Freedom EVO pipetor. The same automate was then used to distribute 2μL of known drug solutions in DMSO (10 mM) in triplicate. Blank wells were also prepared containing DMSO. The solutions were mixed and incubated for one hour at room temperature. The interaction of the drugs with the membrane was recorded by the LSPR shift at 530 nm. The data were averaged and the blank OD due to the DMSO alone was subtracted. A correlation was found with the log of apparent permeation rate of the drugs through Caco-2 cell layers (n=44; r=0.58; p<0.005). Fig 13 shows the correlation obtained for known drugs between the LSPR signal and the Caco-2 permeation rate constant (literature data). A correlation (n= 57; r = 0.78) was observed between the percentage fraction of drugs absorbed in humans in vivo and the LSPR signal corresponding to the extent of drug interaction with the gold nanoparticles surrounded by the artificial membrane. Fig 14 shows the correlation obtained for known drugs between the LSPR signal and the % fraction absorbed in the intestinal tract.

EXAMPLE 10.

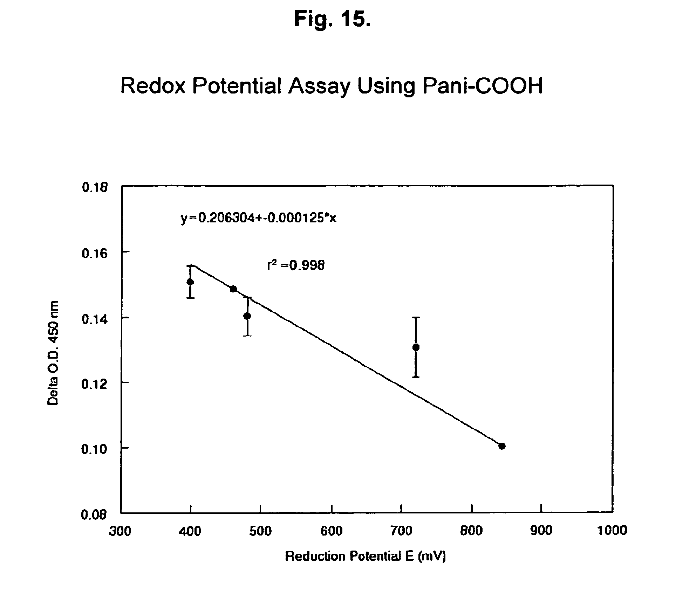

The redox potential of known drugs were assayed. The known drug at a given final concentration (100 μM) was introduced in a buffered solution of a conductive polymer (poly[aniline-2-carboxylic acid], PANI-COOH) at pH 4. The solution was mixed and incubated at room temperature (10 minutes). The optical density was read at 450 nm. A sample blank containing the drug and phtalate buffer was prepared, incubated and read in the same way. The OD 450 nm of the blank solution was subtracted from the OD 450 nm of the conductive polymer reaction mixture. Fig. 15 shows the correlation between the 450 nm signal of PANI-COOH after reaction with known compounds and their respective reduction potentials (literature data). A linear relationship was observed between the 450 nm signal and the reduction potential of the known molecules (Fig. 15.), which allowed the inference of the reduction potential of NCEs.

Screening a library of 60 well-known drugs allowed identification of bromocriptine as a relatively strong oxidant (E = -364 mV), prazosin and chlorpropamide as relatively strong reductants (E = 747 and 848 mV), which is in agreement with literature data (Wardman P. (1989) Reduction potentials of one-electron couples involving free radicals in aqueous solution. J. Phys. Chem. Ref. Data 18, 1637-1755). Although a reductant drug may exert beneficial therapeutic effects, an oxidant may be toxic. The redox potential level of a drug further governs also the kind and rapidity of its metabolism and henceforth modulates its pharmacokinetic profile (Shargel and Yu, 1999, supra).

EXAMPLE 11.

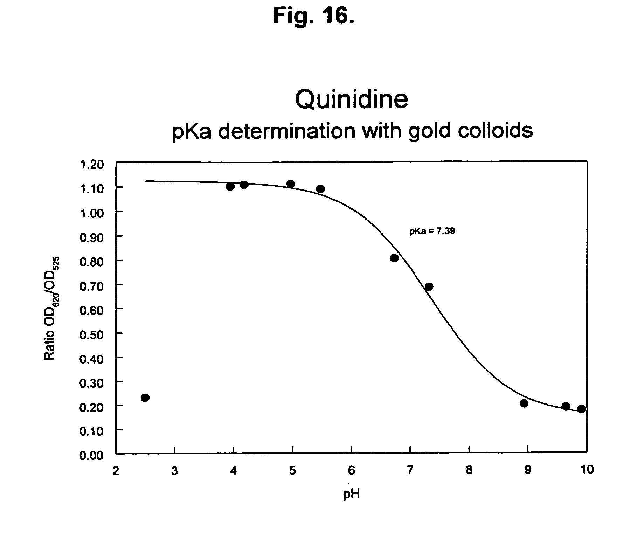

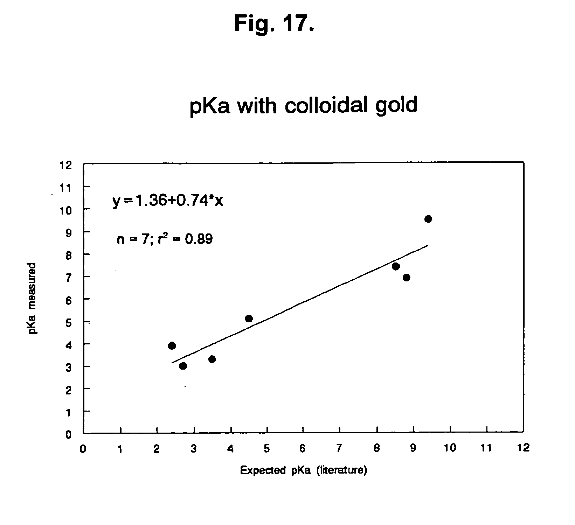

A solution of gold nanoparticles was prepared as described in Example 1. A series of 50mM buffers of pH 2-12 was then prepared (phosphate [pH 2, 3, 6, 7, 8], acetate [pH 4 and 5] and borate [pH 9, 10, 11, 12]), which were further diluted 5-fold with the gold colloid solution. The final pH of the 10 mM buffered colloid so obtained was then recorded. Drug solutions (10 μl, 100 mM in DMSO) were then pipeted in 1 mL of the buffered colloid and the mixture was vortex-mixed. After 30 minutes of incubation, the solutions were pipeted in 96-well polystyrene plates and the O.D. at 620 nm and 525 nm were recorded. The ratio O.D.620 nm/O.D. 525 nm was calculated. This ratio relates to the agglutination of the colloid (i.e., the higher the ratio, the higher the agglutination). Typically, the ionization of a molecule creates charge (s) that may facilitate the agglutination of the colloid. Thus, titering a molecule with buffered gold colloids over a range of pHs can allow one to determine the ionization constant from the interpolated mid-point of the sigmoid curve drawn from agglutinated to unagglutinated colloid as a function of pH. An example of such a titration curve (quinidine) is shown in Fig. 16. Table I below compares results of pKa obtained with the present method with reference values from the literature. Fig. 17 shows the correlation (n = 7; $r^2$ = 0.89) between measured and expected pKa values with colloidal gold.

Table I. Comparison of pKa values obtained by colloidal gold pH titration of 1mM drugs, with reference data from Williams, D.A. and Lemke, T.L. (2002) pKa values for some drugs and miscellaneous organic acids and bases. In: Foye's Principles of Medicinal Chemistry, 5th Edition, Lippincott, Williams and Wilkins, Philadelphia, PA, pp. 1070-1079.

| Drug | pKa observed | pKa from reference |
|---|---|---|
| Aspirin | 3.3 | 3.5 |
| Dapsone | 3.9 | 2.4 |
| Doxycycline | 4.3 (1) | 3.4 (1) |
|  | 8.7 (2) | 9.3 (2) |
| Erythromycine | 6.9 | 8.8 |
| Indomethacine | 5.1 | 4.5 |
| Nafcillin | 3.0 | 2.7 |
| Paracetamol | 9.5 | 9.4 |

(continued)

| Drug | pKa observed | pKa from reference |
|---|---|---|
| Quinidine | 7.4 | 8.5 |

As these data show, the method described in this example allows one to determine the pKa of drugs in a range of pHs. Differences with the reference values are due to the presence of DMSO in the present technique (Bordwell, F.G. (1988) Equilibrium acidities in dimethyl sulfoxide solution. Acc. Chem. Res. 21, 456-463).

It is evident from the above discussion and results that the subject invention provides an important new type of assay that finds use in a variety of different applications for the rapid high-throughput determination of PK/ADME-Tox characteristics of NCEs, drug candidates and lead compounds early in the drug discovery process. For example, use of the subject colloids in the drug discovery and development applications offers an advantage over the radioactive, chromatographic and mass spectrometric techniques in current use that involve a physical separation of the bound from free moieties, because the colloidal conductive polymers, colloidal metals and composite thereof can be applied in homogeneous "mix and read" assays. The assays can be performed in various instruments and for one of ordinary skill in the art, it is evident that many photonic detection systems may be used. The analytical systems of the present invention find use in many aspects of the in vitro PK/ADME-Tox screening of NCEs, drug candidates and lead compounds, such as binding to plasma proteins, membrane interaction, activity toward metabolizing enzymes, hydrophobicity, charge interaction, redox potential, to name a few representative applications. As such, the subject invention represents a significant contribution to the art.

The scope of present invention is embodied by the appended claims.

**Claims**

1. An in vitro method of determining a predicted in vivo ADME/TOX property of a candidate compound, said method comprising:

    (a) contacting said candidate compound with a nanoparticle colloid;
    (b) detecting an optical property of said candidate-compound-contacted nanoparticle colloidal; and
    (c) determining a predicted in vivo ADME/TOX property for said candidate compound from said detected optical property.

2. The method according to Claim 1, wherein an agent is displayed on the surface of said nanoparticle colloid, wherein said agent is selected from the group consisting of: plasma binding proteins, metabolizing enzymes such as members of the cytochrome family, hydrophilic membranes, hydrophobic membranes, detergents, nucleic acids and peptides.

3. The method according to Claim 1, wherein said ADME/TOX property is selected from the group consisting of absorption, distribution, metabolism, excretion, toxicity and redox potential.

4. The method according to Claim 1, wherein said nanoparticle colloid is selected from the group consisting of a conductive polymer colloid, a noble metal colloid and a metal/conductive polymer composite colloid.

5. The method according to Claim 1, wherein said nanoparticle colloid further comprises an agent that interacts with said candidate compound.

6. The method according to Claim 5, wherein said agent is selected from the group consisting of a peptide or a protein, an enzyme, a receptor, and a membrane.

7. The method according to Claim 1, wherein said optical property is light absorbance.

8. The method according to Claim 1, wherein said nanoparticle colloid comprises an agent that specifically binds to said candidate compound and said method further comprises:

    (i) preparing a binding isotherm for binding of said candidate compound to said agent; and
    (ii) estimating the binding affinity of said candidate compound for said agent from said binding isotherm.

9. The method according to Claim 1, wherein said nanoparticle colloid comprises an agent that specifically binds to said candidate compound and said method comprises:

(i) contacting said nanoparticle colloid with a first concentration of said candidate compound to determine association kinetics; and
(ii) contacting said nanoparticle colloid with a second concentration of said candidate compound to determine dissociation kinetics.

10. The method according to Claim 1, wherein said method comprises contacting said candidate compound with said nanoparticle colloid at a plurality of different concentrations and pH values to obtain a redox potential for said candidate compound.

11. The method according to Claim 1, wherein said method is conducted in a high-throughput screening device.

12. The method according to Claim 2, wherein said agent is selected from the group consisting of: thiolated alkyl chains, albumin, $\alpha$1-acid glycoproteins, and cytochrome P450 isozymes.

13. Use of a nanoparticle colloid in an *in vitro* method of determining a predicted *in vivo* ADME/TOX property of a candidate compound.

**Patentansprüche**

1. Eine in-vitro-Methode zur Bestimmung vorhergesagter absorptions-, distributions-, metabolismus- und eliminationsrelevanter sowie toxikologischer Eigenschaften ("ADME/TOX") von Kandidatenverbindungen in lebenden Organismen, welche die folgenden Elemente umfasst:

(a) die Zusammenführung besagter Kandidatenverbindung mit einem Nanopartikelkolloid;
(b) die Ermittlung einer optischen Eigenschaft des Nanopartikelkolloids, das mit besagter Kandidatenverbindung in Berührung gebracht wurde; und
(c) die Bestimmung vorhergesagter ADME/TOX-Eigenschaften in lebenden Organismen besagter Kandidatenverbindung auf der Grundlage der besagten optischen Eigenschaft.

2. Die Methode gemäß Anspruch 1, wobei auf der Oberfläche des besagten Nanopartikelkolloids ein Wirkstoff dargestellt wird, bei dem es sich um einen Angehörigen der folgenden Gruppe handelt: Plasma-bindende Proteine, metabolisierende Enzyme wie die Angehörigen der Cytochromfamilie, hydrophile Membranen, hydrophobe Membranen, Reinigungsmittel, Nukleinsäuren und Peptide.

3. Die Methode gemäß Anspruch 1, wobei es sich bei besagter ADME/TOX-Eigenschaft um eine Angehörige der folgenden Gruppe handelt: Absorption, Distribution, Metabolismus, Elimination, Toxizität und Redoxpotential.

4. Die Methode gemäß Anspruch 1, wobei das besagte Nanopartikelkolloid aus der Gruppe mit den folgenden Angehörigen stammt: leitfähige Polymerkolloide, Edelmetallkolloide und metallische / leitfähige Polymerverbundkolloide.

5. Die Methode gemäß Anspruch 1, wobei das besagte Nanopartikelkolloid ferner einen Wirkstoff enthält, der mit der besagten Kandidatenverbindung interagiert.

6. Die Methode gemäß Anspruch 5, wobei es sich bei dem besagten Wirkstoff um einen Angehörigen der folgenden Gruppe handelt: Peptide oder Proteine, Enzyme, Rezeptoren, Membranen.

7. Die Methode gemäß Anspruch 1, wobei es sich bei der besagten optischen Eigenschaft um Lichtabsorption handelt.

8. Die Methode gemäß Anspruch 1, wobei das besagte Nanopartikelkolloid ferner einen Wirkstoff enthält, der an besagte Kandidatenverbindung bindet und die besagte Methode ferner die folgenden Elemente umfasst:

(i) die Erstellung einer Bindungsisotherme für die Bindung besagter Kandidatenverbindung an den besagten Wirkstoff; und
(ii) die Schätzung der Bindungsaffinität besagter Kandidatenverbindung zu besagtem Wirkstoff für die besagte Bindungsisotherme.

9. Die Methode gemäß Anspruch 1, wobei das besagte Nanopartikelkolloid ferner einen Wirkstoff enthält, der an besagte Kandidatenverbindung bindet und die besagte Methode ferner die folgenden Elemente umfasst:

(i) die Zusammenführung des besagten Nanopartikelkolloids mit einer ersten Konzentration der besagten Kandidatenverbindung zum Zwecke der Bestimmung ihrer Assoziationskinetik; und
(ii) die Zusammenführung des besagten Nanopartikelkolloids mit einer zweiten Konzentration der besagten Kandidatenverbindung zum Zwecke der Bestimmung ihrer Dissoziationskinetik.

10. Die Methode gemäß Anspruch 1, wobei die besagte Methode die Zusammenführung der besagten Kandidatenverbindung mit dem besagten Nanopartikelkolloid unter vielen verschiedenen Konzentrationen und pH-Werten zum Zwecke der Ermittlung eines Redoxpotentials für die besagte Kandidatenverbindung umfasst.

11. Die Methode gemäß Anspruch 1, wobei die besagte Methode in einem Gerät für die Durchführung von Hochdurchsatzanalysen zur Anwendung kommt.

12. Die Methode gemäß Anspruch 2, wobei es sich bei dem besagten Wirkstoff um einen Angehörigen der folgenden Gruppe handelt: thiolatierte Alkylketten, Albumin, $\alpha$-1-Säure-Glycoproteine und Cytochrom-P450-lsozyme.

13. Die Verwendung von Nanopartikelkolloiden für in-vitro-Methoden zur Bestimmung vorhergesagter ADME/TOX-Eigenschaften von Kandidatenverbindungen in lebenden Organismen.

## Revendications

1. Une méthode in vitro de détermination d'une propriété ADME/TOX prédite in vivo d'un composé candidat, ladite méthode comprenant :

(a) la mise en contact du composé candidat avec un nanocolloïde;
(b) la détection d'une propriété optique dudit nanocolloïde mis en contact avec le composé candidat; et
(c) la détermination d'une propriété ADME/TOX prédite in vivo relative au dit composé candidat à partir de ladite propriété optique détectée.

2. La méthode conforme Révendication 1, au sein de laquelle un agent est exposé sur la surface dudit nanocolloïde, et au sein de laquelle ledit agent est sélectionné dans le groupe comprenant: des protéines de liaison du plasma, des enzymes de métabolisation comme les membres de la famille des cytochromes, des membranes hydrophiles, des membranes hydrophobes, des détergents, des acides nucléiques et des peptides.

3. La méthode conforme Révendication 1, au sein de laquelle ladite propriété ADME/TOX est sélectionnée dans le groupe comprenant l'absorption, la distribution, le métabolisme, l'excrétion, la toxicité et le potentiel d'oxydoréduction.

4. La méthode conforme Révendication 1, au sein de laquelle ledit nanocolloïde est sélectionné dans le groupe comprenant un colloïde de polymère conducteur, un colloïde de métal noble et un colloïde composé de métal/polymère conducteur.

5. La méthode conforme Révendication 1, au sein de laquelle ledit nanocolloïde comprend en outre un agent qui interagit avec ledit composant candidat.

6. La méthode conforme Révendication 5, au sein de laquelle ledit agent est sélectionné dans le groupe comprenant un peptide ou une protéine, une enzyme, un récepteur et une membrane.

7. La méthode conforme Révendication 1, au sein de laquelle ladite propriété optique est l'absorbance de la lumière.

8. La méthode conforme Révendication 1, au sein de laquelle ledit nanocolloïde comprend un agent qui se lie de façon spécifique avec le composant candidat, ladite méthode comprenant en outre:

(i) la préparation d'un isotherme de liaison utilisé pour lier ledit composant candidat et ledit agent; et

(ii) l'estimation de l'affinité de liaison dudit composant candidat avec ledit agent à partir dudit isotherme de liaison.

9. La méthode conforme Révendication 1, au sein de laquelle ledit nanocolloïde comprend un agent qui se lie de façon spécifique avec le composant candidat, ladite méthode comprenant:

   (i) la mise en contact dudit nanocolloïde avec la première concentration dudit composant candidat afin de déterminer la cinétique d'association; et
   (ii) la mise en contact dudit nanocolloïde avec la seconde concentration dudit composant candidat afin de déterminer la cinétique de dissociation.

10. La méthode conforme Révendication 1, au sein de laquelle ladite méthode comprend la mise en contact dudit composant candidat avec ledit nanocolloïde à diverses concentrations et valeurs de pH, afin d'obtenir un potentiel d'oxydoréduction pour ledit composant candidat.

11. La méthode conforme Révendication 1, au sein de laquelle ladite méthode est mise en oeuvre par le biais d'un dispositif de criblage à haut débit.

12. La méthode conforme Révendication 2, au sein de laquelle ledit agent est sélectionné dans le groupe comprenant : des chaînes alkyle thiol, des alpha-1 glycoprotéines acides, et des isoenzymes P450 cytochromes.

13. Utilisation d'un nanocolloïde dans une méthode *in vitro* de détermination d'une propriété ADME/TOX prédite *in vivo* d'un composé candidat.

**Fig. 1.**

Theophylline interaction with BSA

# Fig. 2.

## Bilirubin interaction with BSA

# Fig. 3.

Titration of phosphate-buffered poly(aniline)

# Fig. 4.

Kinetics of Association and Dissociation of hSA-Digoxine

# Fig. 5.

## Ascorbic acid standard curve

# Fig. 6.

## Volume of Distribution

$y = 0.185 + 0.80x$
$r^2 = 0.96$

Volume of Distribution in vitro, L/Kg

Volume of Distribution in vivo, L/Kg

# Fig. 7.

## Digoxin-Human Serum Albumin

# Fig. 8.

## hSA Binding Isotherm (Thiopental)

$r^2 = 0.92$

$K_D = 43.9\ \mu M$
$K_A = 2.3\ 10^4\ L.mol^{-1}$

ΔOD 530nm

Thiopental added μM

# Fig. 9.

## Assay Reproducibility

# Fig. 10.

## Correlation Between Observed and Expected Apparent Affinities (log)

$y = 0.8 + 0.9 \cdot x$

$r^2 = 0.75; n = 55$

Log apparent Ka observed (L.mol$^{-1}$)

Log apparent Ka expected (L.mol$^{-1}$)

**Fig. 11.**

Orosomucoid Binding Isotherms (Verapamil and
Erythromycine)

# Fig. 12.

## Orosomucoid Correlation with Literature Data and Reproducibility

# Fig. 13.

# Correlation between LSPR Signal of Known Drugs and Caco-2 Permeation Rate Constant (literature data)

$y = -5.39 + 9.88 \cdot x$

$n = 44; r = 0.58$

Log Permeation rate Caco-2 $10^{-4}$ cm.sec$^{-1}$

# Fig. 14.

# Correlation between LSPR Signal of Known Drugs and Percentage Fraction Absorbed by Oral Administration (literature data)

y = 84.85+0.94*x

n = 57; r = 0.78

ΔmAU 530 nm versus blank (100 pM)

# Fig. 15.

## Redox Potential Assay Using Pani-COOH

y=0.206304+-0.000125*x

r$^2$ =0.998

Delta O.D. 450 nm

Reduction Potential E (mV)

# Fig. 16.

## Quinidine
### pKa determination with gold colloids

pKa = 7.39

# Fig. 17.

## pKa with colloidal gold

y = 1.36+0.74*x

n = 7; $r^2$ = 0.89

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004009536 A
- US 2005119832 A
- WO 9942511 A
- WO 03027656 A
- WO 2005034915 A
- US 6868343 B
- US 20070172834 A
- US 6395561 B
- US 5948902 A
- WO 2006047371 A, Englebienne and Van Hoonacker
- WO 2006 A
- US 6127133 A

**Non-patent literature cited in the description**

- **Bains, W.** Failure rates in drug discovery and development: Will we ever get any better?. *Drug Discovery World,* vol. 5 (4), 9-18
- **Englebienne, P.** High-Throughput Screening: Will the past meet the future?. *Frontiers in Drug Design and Discovery,* vol. 1, 69-86
- **Service, R.F.** Surviving the blockbuster syndrome. *Science,* 2004, vol. 303, 1796-1799
- **Wang, J. ; Urban, L.** The impact of early ADME profiling on drug discovery and development strategy. *Drug Discovery World,* 2004, vol. 5 (4), 73-86
- **Yoshida, F. ; Topliss, J.G.** QSAR model for drug human oral bioavailability. *J. Med. Chem.,* 2000, vol. 43, 2575-2585
- **Bergström, C.A.S. ; Strafford, M. et al.** Absorption classification of oral drugs based on molecular surface properties. *J. Med. Chem.,* 2003, vol. 46, 558-570
- **Sköld, C. ; Winiwater, S. et al.** Presentation of a structurally diverse and commercially available drug data set for correlation and benchmarking studies. *J. Med. Chem.,* 2006, vol. 49, 6660-6671
- **Lombardo, F. ; Obach, R.S. et al.** Prediction of human volume of distribution values for neutral and basic drugs. 2. Extended data set and leave-class-out statistics. *J. Med. Chem.,* 2004, vol. 47, 1242-1250
- **Wilson, D.M. ; Wang, X. et al.** High-throughput Log D determination using liquid chromatography-mass spectrometry. *Combinatorial Chem. High Throughput Screen,* 2001, vol. 4, 511-519
- **Taillardat-Bertschinger, A. ; Carrupt, P.A. et al.** Immobilized artificial membrane HPLC in drug research. *J. Med. Chem.,* 2003, vol. 46, 655-665
- **Cimitan, S. ; Lindgren, M.T. et al.** Early absorption and distribution analysis of antitumor and anti-AIDS drugs: lipid membrane and plasma protein interactions. *J. Med. Chem.,* 2005, vol. 48, 3536-3546
- **Englebienne, P. et al.** Surface plasmon resonance: Principles, methods and applications in biomedical sciences. *Spectroscopy,* 2003, vol. 17, 255-278
- **Bao, Y.P. ; Wei, T.F. ; Lefebvre, P.A. et al.** Detection of protein analytes via nanoparticle-based bio bar code technology. *Anal. Chem.,* 2006, vol. 78, 2055-2059
- **Englebienne, P.** Synthetic materials capable of reporting biomolecular recognition events by chromic transition. *J. Mater. Chem.,* 1999, vol. 9, 1043-1054
- **Englebienne, P. ; Van Hoonacker, A.** Gold-conductive polymer nanoparticles: A hybrid material with enhanced photonic reactivity to environmental stimuli. *J. Coll. Interface Sci.,* 2005, vol. 292, 445-454
- **Englebienne, P.** Immune and Receptor Assays in Theory and Practice. CRC Press, 2000, vol. 392pp, 243, 234
- **Peeters, T.Jr.** All about Albumin. *All about Albumin,* 1996, vol. 432pp, 97
- **Goodman ; Gillman et al.** The Pharmacological Basis of Therapeutics. McGraw-Hill, 1996
- **Lombardo, F. ; Shalaeva, M.Y. et al.** ElogPoct: A tool for lipophilicity determination in drug discovery. *J. Med. Chem.,* 2000, vol. 43, 2922-2928
- **Westphal, U.** Steroid-Protein Interactions II. Springer-Verlag, 1986, vol. 603pp
- **Shargel, L. ; Yu, A.B.C.** Applied Biopharmaceutics and Pharmacokinetics. McGraw-Hill, 1999, vol. 768pp

- **Wardman P.** Reduction potentials of one-electron couples involving free radicals in aqueous solution. *J. Phys. Chem. Ref. Data,* 1989, vol. 18, 1637-1755

- **Bordwell, F.G.** Equilibrium acidities in dimethyl sulfoxide solution. *Acc. Chem. Res.,* 1988, vol. 21, 456-463